(19) Europäisches Patentamt
European Patent Office
Office européen des brevets

(11) Veröffentlichungsnummer : **0 336 886 B1**

(12) **EUROPÄISCHE PATENTSCHRIFT**

(45) Veröffentlichungstag der Patentschrift :
**26.08.92 Patentblatt 92/35**

(51) Int. Cl.$^5$ : **C07D 333/64,** C07D 409/06, A61K 31/38

(21) Anmeldenummer : **89810170.4**

(22) Anmeldetag : **07.03.89**

(54) **Thioverbindungen.**

(30) Priorität : **16.03.88 CH 998/88**

(43) Veröffentlichungstag der Anmeldung :
**11.10.89 Patentblatt 89/41**

(45) Bekanntmachung des Hinweises auf die Patenterteilung :
**26.08.92 Patentblatt 92/35**

(84) Benannte Vertragsstaaten :
**AT BE CH DE ES FR GB GR IT LI LU NL SE**

(56) Entgegenhaltungen :
**EP-A- 0 078 240**
**FR-M- 5 822**
**US-A- 4 663 344**

(56) Entgegenhaltungen :
**CHEMICAL ABSTRACTS, Band 78, Nr. 21, 28.05.1973, Seite 355, Zusammenfassung Nr. 135981b, Columbus, Ohio, USA; N.0. VESTERAGERet al.: "Thiophene chemistry. XXII. Reactions of benzo(b)thiophen-2(3H)-one"; & Tetrahedron 1973, 29/2), 321-329 in Verbindungmit CHEMICAL ABSTRACTS, Band 78, 1973, Formelindex, C16-Z, Seite 1245F, linke Spalte, Zeilen 12-14**

(73) Patentinhaber : **CIBA-GEIGY AG**
**Klybeckstrasse 141**
**CH-4002 Basel (CH)**

(72) Erfinder : **Ferrini, Pier Giorgio, Dr.**
**Im Rehwechsel 22**
**CH-4102 Binningen (CH)**

EP 0 336 886 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Benzo[b]thiophen-Derivate der Formel

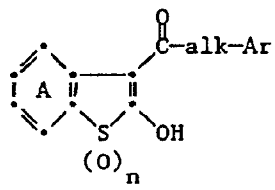

(I),

ihre Tautomeren und Salze, worin der Index n 0, 1 oder 2 bedeutet, alk Niederalkylen bedeutet, Ar Phenyl, Naphthyl oder einen monocyclischen, fünf- oder sechsgliedrigen Heteroarylrest bedeutet, wobei der aromatische Rest Ar unsubstituiert oder ein- oder mehrfach durch Niederalkyl, Halogenniederalkyl, Halogen und/oder Nitro substituiert ist, und der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist, Verfahren zu ihrer Herstellung, ihre Verwendung und diese Verbindungen enthaltende pharmazeutische Präparate sowie deren Herstellung.

Die freien Verbindungen der Formel I können in einem dynamischen Gleichgewicht mit entsprechenden Tautomeren davon stehen. Die 2-Hydroxy-benzo[b]thiophen-Derivate der Formel I können somit als 2-Oxo-2,3-dihydro-benzo[b]thiophen-Verbindungen der Formel Ia

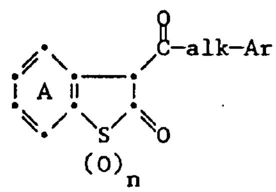

(Ia)

vorliegen, wobei jedoch das Gleichgewicht überwiegend auf Seiten der entsprechenden 2-Hydroxy-benzo[b]thiophen-Tautomeren liegt.

Die Verbindungen der Formeln I und Ia können auch Salze bilden, insbesondere Salze mit Basen, wobei diese Salzbildung mit der aciden Sauerstoffunktion in Position 2 des Benzo[b]thiophenrings erfolgt. Entsprechende Salze sind z.B. Metallsalze, wie Alkalimetall- oder Erdalkalimetall-, z.B. Natrium-, Kalium-, Magnesium- oder Calciumsalze, Aluminium- oder Uebergangsmetallsalze, wie Zink- oder Kupfersalze, oder entsprechende Salze mit Ammoniak oder organischen Aminen. Als organische Amine kommen z.B. die folgenden in Betracht: Alkylamine, wie Mono-, Di-oder Triniederalkylamine, Alkylendiamine, wie Niederalkylendiamine, durch Phenyl substituierte Alkylamine, wie Mono- oder Di-phenylniederalkylamine, Hydroxyalkylamine, wie Mono-, Di- oder Trihydroxyniederalkylamine, ein Oligohydroxyniederalkylamin oder Hydroxyniederalkyl-diniederalkylamin, Aminozucker, z.B. solche, deren Aminogruppe gegebenenfalls durch mindestens einen Niederalkylrest substituiert sein kann, Cycloalkylamine, wie Mono- oder Di-cycloniederalkyl-amine, basische Aminosäuren, cyclische Amine, wie Niederalkylen- oder Niederalkenylenamine mit 2 bis 6 C-Atomen, wobei die Kohlenstoffkette auch durch Aza, N-Niederalkyl-aza, Oxa und/oder Thia unterbrochen sein kann. Mono-, Di- oder Triniederalkylamine sind z.B. Ethyl- oder tert.-Butylamin, Diethyl oder Diisopropylamin, Trimethyl-oder Triethylamin, und Niederalkylendiamin ist z.B. Ethylendiamin. Als Phenylniederalkyl-amine kommen z.B. Benzyl- oder 1- oder 2-Phenylethyl-amin in Betracht. Mono-, Di- oder Trihydroxyniederalkyl-amine sind z.B. Mono-, Di-, Triethanolamin oder Diisopropanol-amin, ein Oligohydroxyniederalkyl-amin z.B. Tris-(hydroxymethyl)-methyl-amin und Hydroxyniederalkyl-diniederalkylamine z.B. N,N-Dimethyl- oder N,N-Diethylamino-ethanol. Aminozucker leiten sich z.B. von Monosacchariden ab, bei denen eine alkoholische Hydroxygruppe durch eine Aminogruppe ersetzt ist, wie D-Glucosamin, D-Galaktosamin oder Marmosamin. Als Beispiel für einen N-niederalkylierten Aminozucker sei N-Methyl-D-glucosamin genannt. Mono- oder Di-cycloniederalkyl-amin ist z.B. Cyclohexyl- oder Dicyclohexylamin. Basische Aminosäuren sind z.B. Arginin, Histidin, Lysin oder Ornithin. Niederalkylen- bzw. Niederalkenylen-amine sind z.B. Azirin, Pyrrolidin, Piperidin oder Pyrrolin und als Niederalkylen- bzw. Niederalkenylenamine, deren Kohlenstoffkette durch Aza, N-Niederalkylaza, Oxa und/oder Thia unterbrochen ist,

kommen z.B. Imidazolin, 3-Methyl-imidazolin, Piperazin, 4-Methyl- oder 4-Ethylpiperazin, Morpholin oder Thiomorpholin in Frage. Bevorzugt sind pharmazeutisch verwendbare Salze, z.B. Alkalimetall-, wie Natriumsalze.

Die Variable alk steht insbesondere für -CH(R)-, wobei R Niederalkyl oder Wasserstoff bedeutet.

Monocyclische fünfgliedrige Heteroarylreste sind z.B. entsprechende monoaza-, diaza-, triaza-, tetraaza-, monooxa-, monothia-, oxaza-, oxadiaza-, thiaza- oder thiadiazacyclische Reste, wie Pyrrolyl, Pyrazolyl, Imidazolyl, Triazolyl, Tetrazolyl, Furyl, Thienyl, Isoxazolyl, Oxazolyl, Oxadiazolyl, Isothiazolyl, Thiazolyl oder Thiadiazolyl.

Monocyclische sechsgliedrige Heteroarylreste stellen z.B. entsprechende monoaza-, diaza- oder triazacyclische Reste dar, wie Pyridyl, Pyridazinyl, Pyrimidinyl, Pyrazinyl oder Triazinyl, wobei Pyridylreste auch als 1-Oxido-pyridyle vorliegen können.

In entsprechenden fünf- oder sechsgliedrigen Heteroarylresten, die eine -NH-Gruppe aufweisen, kann das betr. Wasserstoffatom durch Niederalkyl substituiert sein. Als Beispiele für derartige Reste Ar seien 1-Niederalkyl-pyrrolyl, wie 1-Methyl-pyrrol-2-yl, oder 1-Niederalkyl-imidazolyl, wie 1-Methyl-imidazol-2-yl, genannt.

Der Ring A bzw. der Rest Ar kann insbesondere unsubstituiert oder einfach, ferner auch mehrfach, z.B. zwei- oder dreifach, substituiert sein.

Die vor- und nachstehend verwendeten Allgemeindefinitionen haben, sofern nicht abweichend definiert, in erster Linie die folgenden Bedeutungen:

Der Ausdruck "Nieder" bedeutet, dass entsprechende Gruppen oder Verbindungen insbesondere bis und mit 7, vorzugsweise bis und mit 4, Kohlenstoffatome enthalten.

Niederalkylen ist geradkettig oder verzweigt und bedeutet beispielsweise Methylen, Ethylen, 1-Methyl-methylen, ferner 1- oder 2-Methyl-ethylen, Propylen oder Butylen.

Niederalkyl ist z.B. Methyl, Ethyl, n-Propyl, Isopropyl, n-Butyl, Isobutyl, sek.-Butyl, tert.-Butyl und umfasst ferner entsprechende Pentyl-, Hexyl- und Heptylreste.

Halogenniederalkyl ist z.B. Trifluormethyl, 1,1,2-Trifluor-2-chlorethyl oder Chlormethyl.

Halogen ist insbesondere Halogen mit Atomnummer bis und mit 35, wie Fluor, Chlor oder Brom, und umfasst ferner Iod.

Niederalkoxy ist z.B. Methoxy, Ethoxy, n-Propyloxy, Isopropyloxy, n-Butyloxy, Isobutyloxy, sek.-Butyloxy, tert.-Butyloxy und umfasst ferner entsprechende Pentyloxy-, Hexyloxy- und Heptyloxyreste.

Niederalkylthio ist z.B. Methyl-, Ethyl-, n-Propyl-, Isopropyl-, n-Butyl-, Isobutyl-, sek.-Butyl- oder tert.-Butylthio.

Niederalkansulfinyl bzw.-sulfonyl ist z.B. Methan-, Ethan-, n-Propan- oder Isopropan-sulfinyl bzw. -sulfonyl.

Die Verbindungen der Formel I bzw. Ia sowie ihre pharmazeutisch verwendbaren Salze weisen z.B. wertvolle pharmakologische Eigenschaften auf. So besitzen sie vor allem ausgeprägte analgetische Eigenschaften, die sich z.B. aus der Reduktion des durch Phenyl-p-benzochinon induzierten Writhing-Syndroms an der Maus ab einer Dosis von etwa 0,1 mg/kg p.o. ableiten lassen [Methodik: L.C. Hendershot and J. Forsaith, J. Pharmacol. exp. Ther. 125, 237 ff. (1959) sowie A. Schweizer et al., Agents and Actions 23, 1/2 (1988) in press]. Ebenso erzeugen die erfindungsgemässen Verbindungen in der Versuchsanordnung gemäss Pain Res. and Therap., Vol. 1, 517 ff. (1976), Raven Press N.A. eine Hemmung des durch Essigsäure ausgelösten Writhing-Syndroms an der Ratte ab einer Dosis von etwa 1 mg/kg p.o. Die analgetische Aktivität manifestiert sich vorteilhaft über einen längeren Zeitabschnitt, so dass eine Dosierung weniger häufig erfolgen kann.

Weiterhin weisen die erfindungsgemässen Verbindungen eine überraschend günstige gastrointestinale Verträglichkeit auf, was wiederum eine hohe therapeutische Breite zur Folge hat. So wurde gemäss der Methodik von I. Böttcher et al., Drugs exp. clin. Res. 13, 237 ff. (1987) der gastrointestinale Blutverlust bei der Ratte während 10-tägiger Behandlung mit den Wirksubstanzen untersucht. Dabei hat sich gezeigt, dass erst in einem Dosisbereich von etwa 50 bis 200 mg/kg p.o. eine leichte Zunahme der Blutungsneigung festzustellen ist, die über das Mass hinausgeht, das bei unbehandelten Ratten zu beobachten ist.

Weiterhin weisen die erfindungsgemässen Verbindungen ausgeprägte hemmende Eigenschaften auf Cyclooxygenase und gleichzeitig 5-Lipoxygenase auf. Hemmer dieser Enzyme können zur Behandlung von entzündlichen Prozessen verwendet werden.

Dementsprechend können die Verbindungen der Formeln I und Ia sowie ihre pharmazeutisch verwendbaren Salze z.B. als (periphere) Analgetika und Antiinflammatorika, zur Behandlung von Schmerzzuständen und endzündlicher Prozesse verwendet werden. Ein weiterer Gegenstand der Erfindung ist die Verwendung der erfindungsgemässen Verbindungen zur therapeutischen und/oder prophylaktischen Behandlung des menschlichen, ferner tierischen, Körpers, insbesondere zur Behandlung von Schmerzzuständen, sowie die Verwendung zur Herstellung von Arzeinmitteln, insbesondere von Analgetika. Dabei ist auch die gewerbsmässige Herrichtung der Wirksubstanzen eingeschlossen.

Die Erfindung betrifft insbesondere Verbindungen der Formeln I und Ia sowie ihre Salze, worin der Index

n 0, 1 oder 2 bedeutet, alk für Niederalkylen, insbesondere für -CH(R)- und R für Wasserstoff oder Niederalkyl steht, Ar jeweils unsubstituiertes oder ein- oder mehrfach durch Niederalkyl, Halogenniederalkyl, Halogen und/oder Nitro substituiertes Phenyl, Naphthyl, Pyrrolyl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, wobei in entsprechenden Heteroarylresten, die die -NH-Gruppe aufweisen, das betreffende Wasserstoffatom durch Niederalkyl substituiert sein kann, und der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist.

Die Erfindung betrifft insbesondere Verbindungen der Formeln I und Ia sowie ihre Salze, worin der Index n 0, 1 oder 2 bedeutet, Ar jeweils unsubstituiertes oder ein- oder mehrfach durch Niederalkyl, Halogenniederalkyl, Halogen und/oder Nitro substituiertes Phenyl, Naphthyl, Pyrrolyl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist, und alk für -CH(R)-steht, wobei R Wasserstoff oder Niederalkyl bedeutet.

Die Erfindung betrifft insbesondere Verbindungen der Formeln I und Ia sowie ihre Salze, worin der Index n für 0, ferner 1 oder 2 steht, alk für Niederalkylen, insbesondere für -CH(R)- und R für Wasserstoff oder Niederalkyl steht, Ar unsubstituiertes oder ein- oder mehrfach durch Niederalkyl, Niederalkoxy, Halogenniederalkyl, insbesondere Trifluoromethyl, oder Halogen substituiertes Phenyl, Pyrrolyl, 1-Niederalkylpyrrolyl, Thienyl, Thiazolyl oder Pyridyl bedeutet und der Ring A unsubstituiert oder ein- oder mehrfach durch Niederalkyl, Niederalkoxy, Halogenniederalkyl, insbesondere Trifluormethyl, Halogen oder Nitro substituiert ist.

Die Erfindung betrifft insbesondere Verbindungen der Formeln I und Ia sowie ihre Salze, worin der Index n für 0, 1 oder 2 steht, alk für -CH(R)- steht und R Wasserstoff oder Niederalkyl bedeutet, Ar unsubstituiertes oder ein- oder mehrfach durch Halogenniederalkyl, insbesondere Trifluormethyl, oder Halogen substituiertes Phenyl, Pyrrolyl, Thienyl oder Pyridyl bedeutet und der Ring A unsubstituiert oder ein- oder mehrfach durch Niederalkyl, Niederalkoxy, Halogenniederalkyl, insbesondere Trifluormethyl, Halogen oder Nitro substituiert ist.

Die Erfindung betrifft insbesondere Verbindungen der Formeln I und Ia sowie ihre Salze, worin der Index n 0 bedeutet, alk für -CH(R)- steht und R Niederalkyl, insbesondere mit bis und mit 4 C-Atomen, wie Methyl, bedeutet, Ar unsubstituiertes oder durch Niederalkoxy, insbesondere mit bis und mit 4 C-Atomen, wie Methoxy, Halogen, insbesondere mit Atomnummer bis und mit 35, wie Fluor oder Chlor, oder Trifluormethyl, jeweils insbesondere in Position 3 oder 4 lokalisiert, substituiertes Phenyl, Thiazolyl, wie 2-Thiazolyl, oder Pyridyl, wie 2-Pyridyl, oder unsubstituiertes 1-Niederalkyl-pyrrolyl, insbesondere mit bis und mit 4 C-Atomen im Niederalkylteil, wie 1-Methyl-pyrrol-2-yl, oder unsubstituiertes oder durch Niederalkyl, insbesondere mit bis und mit 4 C-Atomen, wie Methyl, oder Halogen, insbesondere mit Atomnummer bis und mit 35, wie Brom, substituiertes Thienyl, wie 2- oder 3-Thienyl, bedeutet und der Ring A unsubstituiert oder durch Halogen, insbesondere mit Atomnummer bis und mit 35, wie Fluor oder Chlor, oder Nitro, insbesondere in Position 5 oder 6 des Ringsystems, substituiert ist.

Die Erfindung betrifft insbesondere Verbindungen der Formel I und Ia sowie ihre Salze, worin der Index n 0, ferner 1 oder 2 bedeutet, alk für -CH(R)-steht und R Wasserstoff und Niederalkyl, insbesondere mit bis und mit 4 C-Atomen, bedeutet, Ar unsubstituiertes oder durch Halogenniederalkyl, insbesondere mit bis und mit 2 C-Atomen, wie Trifluormethyl, oder Halogen, insbesondere mit Atomnummer bis und mit 35, wie Fluor oder Chlor, substituiertes Phenyl, oder unsubstituiertes Pyrrolyl, Thienyl oder Pyridyl bedeutet und der Ring A unsubstituiert oder durch Halogen, insbesondere mit Atomnummer bis und mit 35, wie Fluor oder Chlor, oder Nitro monosubstituiert ist.

Die Erfindung betrifft in erster Linie Verbindungen der Formeln I und Ia sowie ihre Salze, worin der Index n 0 ist, alk für -CH(R)- steht und R Wasserstoff ist, Ar unsubstituiertes oder durch Trifluormethyl oder Halogen mit Atomnummer bis und mit 35, wie Fluor, insbesondere in Position 3 oder 4, substituiertes Phenyl oder unsubstituiertes Thienyl, wie 2-Thienyl, bedeutet und der Ring A unsubstituiert oder durch Halogen mit Atomnummer bis und mit 35, wie Fluor, insbesondere in Position 5 oder 6 des Ringsystems, substituiert ist.

Die Erfindung betrifft in erster Linie Verbindungen der Formeln I und Ia sowie ihre Salze, worin der Index n 0 ist, alk für -CH(R)- und R für Wasserstoff steht, Ar Phenyl oder 2- oder 3-Thienyl bedeutet und der Ring A unsubstituiert oder in Position 5 oder 6 des Ringsystems durch Fluor oder Chlor substituiert ist.

Die Erfindung betrifft in erster Linie Verbindungen der Formeln I und Ia sowie ihre Salze, worin der Index n 0 ist, alk für -CH(R)-steht und R Wasserstoff ist, Ar Thienyl, wie 2-Thienyl, bedeutet und der Ring A unsubstituiert oder durch Fluor, insbesondere in Position 5 oder 6 des Ringsystems, substituiert ist.

Die Erfindung betrifft insbesondere die in den Beispielen genannten neuen Verbindungen und ihre Herstellung.

Verfahren zur Herstellung der erfindungsgemässen Verbindungen sind ebenfalls Gegenstand der Erfindung. Die Herstellung von Verbindungen der Formel I, ihrer Tautomeren und ihrer Salze erfolgt in an sich bekannter Weise und ist z.B. dadurch gekennzeichnet, dass man

a) eine Verbindung der Formel

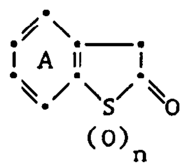

(IIa),

ein Tautomeres oder Salz davon mit einer Verbindung der Formel $X_1$-CO-alk-Ar (IIb), worin $X_1$ reaktionsfähiges verestertes Hydroxy bedeutet, umsetzt oder

b) eine Verbindung der Formel

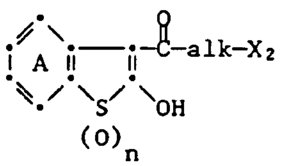

(IIIa),

ein Tautomeres oder Salz davon, worin $X_2$ reaktionsfähiges verestertes Hydroxy bedeutet, mit einer Verbindung der Formel Ar-H (IIIb) umsetzt

und gewünschtenfalls eine verfahrensgemäss oder auf andere Weise erhältliche Verbindung in eine andere Verbindung der Formel I bzw. Ia überführt, ein verfahrensgemäss erhältliches Isomerengemisch in die Komponenten auftrennt, eine verfahrensgemäss erhältliche freie Verbindung der Formel I bzw. Ia in ein Salz überführt und/oder ein verfahrensmäss erhältliches Salz in die freie Verbindung der Formel I bzw. Ia oder in ein anderes Salz überführt.

Die vor- und nachstehend in den Varianten beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -78°C bis zur Siedetemperatur des Reaktionsmediums, vorzugsweise von etwa -10°C bis etwa 150°C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial der Formeln IIa und IIb sowie IIIa und IIIb, das für die Herstellung der Verbindungen der Formel I, deren Tautomeren und deren Salzen entwickelt wurde, ist zum Teil bekannt oder kann ebenfalls nach an sich bekannten Methoden, z.B. analog den vorstehend beschriebenen Verfahrensvarianten, hergestellt werden.

Freie Verbindungen der Formeln IIa und IIIa können auch in Form der entspechenden Tautomeren vorliegen, z.B. Verbindungen der Formel IIa in Form der entsprechenden 2-Hydroxy-benzo[b]thiophene und Verbindungen der Formel IIIa in Form der entsprechenden 2-Oxo-2,3-dihydro-benzo[b]thiophen-Tautomeren, wobei z.B. im Fall von Verbindungen der Formel IIa das Gleichgewicht bevorzugt auf Seiten der 2-Oxo- und bei Verbindungen der Formel IIIa das Gleichgewicht bevorzugt auf Seiten der 2-Hydroxy-benzo[b]thiophen-Form liegt.

Die Ausgangsmaterialien der Formeln IIa und IIIa können z.B. Salze mit Basen bilden, z.B. solche der im Zusammenhang mit den Verbindungen der Formel I aufgeführten Art, wobei die Salzbildung mit der aciden Sauerstoffunktion in Position 2 des Ringsystems erfolgt.

Im Rahmen der vor- und nachstehenden Verfahrensbeschreibung bedeutet reaktionsfähiges verestertes Hydroxy, z.B. $X_1$, sofern nicht abweichend definiert, insbesondere mit einer starken anorganischen Säure, organischen Sulfonsäure, organischen Carbonsäure oder Kohlensäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan-oder Trifluormethansulfonyloxy, $C_3$-$C_7$-Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl-oder p-Toluolsulfonyloxy, gegebenenfalls, z.B. durch Halogen, substituiertes Niederalkanoyloxy, wie Acetyl- oder Trifluoracetyloxy, gegebenenfalls, z.B. durch Niederalkyl, Niederalkoxy, Halogen und/oder Nitro, substituiertes Benzoyloxy, oder Niederalkoxycarbonyloxy, wie Isobutyloxycarbonyloxy.

Variante a):

$X_1$ steht in erster Linie für Halogen, wie Chlor.

Die verfahrensgemässe Umsetzung wird in an sich bekannter Weise durchgeführt, insbesondere in Gegenwart einer Base.

Als Basen kommen beispielsweise Alkalimetallhydroxide, -hydride, -amide, -alkanolate, -carbonate, -triphenylmethylide, -di-$C_1$-$C_7$-alkylamide, -amino-$C_1$-$C_7$-alkylamide oder -$C_1$-$C_7$-alkylsilylamide, Naphthalinamine, $C_1$-$C_7$-Alkylamine, basische Heterocyclen, Ammoniumhydroxide sowie carbocyclische Amine in Frage. Beispielhaft seien Lithiumhydroxid, Natriumhydroxid, -hydrid, -amid, -ethylat, Kalium-tert-butylat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triethylamin, Pyridin, Benzyl-trimethyl-ammoniumhydroxid, 1,5-Diaza-bicyclo[4.3.0]non-5-en (DBN) sowie 1,8-Diaza-bicyclo[5.4.0]undec-7-en (DBU) genannt.

Die in dieser Verfahrensvariante zu verwendenden Ausgangsstoffe sind z.T. bekannt oder können in an sich bekannter Weise hergestellt werden.

Variante b):

$X_2$ bedeutet insbesondere Halogen, wie Chlor, sowie Niederalkoxycarbonyloxy, wie Isobutyloxycarbonyloxy.

Die Alkylierung von Verbindungen der Formel IIIb wird nach an sich bekannter Methodik durchgeführt, insbesondere in Gegenwart einer Lewissäure oder einem Addukt, wie Etherat, davon. Als Lewissäuren kommen beispielsweise Halogenide von Bor, Phosphor, Arsen, Antimon, Zinn, Silber, Zink oder Eisen in Betracht, wie $BF_3$, $AlCl_3$, $FeCl_3$.

Die in dieser Verfahrensvariante zu verwendenden Ausgangsmaterialien der Formel IIIa können in an sich bekannter Weise hergestellt werden. So kann beispielsweise eine Verbindung der Formel

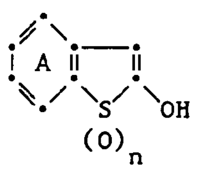

(IIa),

ein Tautomeres oder Salz davon mit einer Verbindung der Formel $X_1$-CO-alk-$X_2$ (IIIc), worin $X_1$ und $X_2$ die vorstehend angegebenen Bedeutungen haben, gemäss der in Variante a) beschriebenen Methode umgesetzt werden.

Die in dieser Verfahrensvariante zu verwendenden Ausgangsstoffe der Formel IIIb sind bekannt oder können in an sich bekannter Weise hergestellt werden.

Die Erfindung betrifft ebenfalls die nach den vorstehenden Verfahrensvarianten erhältlichen neuen Verbindungen.

Eine erfindungsgemäss oder auf andere Weise erhältliche Verbindung der Formel I kann in an sich bekannter Weise in eine andere Verbindung der Formel I übergeführt werden.

Thio kann man z.B. auf übliche Weise zu entsprechendem Sulfinyl bzw. Sulfonyl oxidieren. Als geeignete Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig-, p-Nitroperbenzoe-, m-Chlorperbenzoe- bzw. Perbenzoesäure oder p-Toluolpersulfonsäure, oder Gemische aus Wasserstoffperoxid und Säuren, z.B. Gemische aus Wasserstoffperoxid mit Essigsäure, in Betracht. Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Uebergangsmetalloxide, wie Oxide von Elementen der V. oder VI. Nebengruppe, z.B. Vanadium-, Molybdän-oder Wolframoxid, zu nennen sind. Die Oxidation wird unter milden Bedingungen, z.B. bei Temperaturen von etwa -50° bis etwa +100°C, durchgeführt. Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation von Thio zu Sulfonyl. Jedoch setzt man hierbei üblicherweise das Oxidationsmittel im Ueberschuss ein.

Salze der Formel (I) können in an sich bekannter Weise hergestellt werden. So erhält man beispielsweise Salze mit Basen von freien Verbindungen der Formel (I) durch Behandeln mit einer Base oder einem geeigneten Ionenaustauscherreagenz. Salze können in üblicher Weise in die freien Verbindungen überführt werden,

Salze mit Basen z.B. durch Behandeln mit einem geeigneten sauren Mittel.

Je nach Verfahrensweise bzw. Reaktionsbedingungen können die erfindungsgemässen Verbindungen mit salzbildenden, insbesondere aciden, Eigenschaften in freier Form oder in Form von Salzen oder als Tautomere erhalten werden.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung oder die entsprechenden Tautomeren zu verstehen.

Die neuen freien Verbindungen einschliesslich ihrer Salze können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate aufgetrennt werden, beispielsweise durch fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Ueberführung in diastereomere Salze, z.B. durch Umsetzung eines aciden Endstoffracemats mit einer optisch aktiven Base, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, beispielsweise der Formeln IIa, IIb, IIIa und IIIb die speziell für die Herstellung der erfindungsgemässen Verbindungen entwickelt wurden, ihre Verwendung und Verfahren zu ihrer Herstellung bilden ebenfalls einen Gegenstand der Erfindung, wobei die Variablen die für die jeweils bevorzugten Verbindungsgruppen der Formel I angegebenen Bedeutungen haben.

Die Erfindung betrifft ebenfalls die Verwendung der Verbindungen der Formel (I) bzw. (Ia) oder von pharmazeutisch verwendbaren Salzen von solchen Verbindungen mit salzbildenden Eigenschaften, insbesondere als pharmakologische, in erster Linie analgetisch wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Analgetika, z.B. zur Behandlung von Schmerzzuständen, verwenden.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung oder pharmazeutisch verwendbare Salze davon enthalten, handelt es sich um solche zur enteralen, wie oralen, ferner rektalen, und parenteralen sowie topischen Verabreichung an Warmblüter(n), wobei der pharmakologische Wirkstoff allein oder zusammen mit einem pharmazeutisch anwendbaren Trägermaterial enthalten ist.

Die neuen pharmazeutischen Präparate enthalten z.B. von etwa 10 % bis etwa 80 %, vorzugsweise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung von Mais-, Weizen-, Reis- oder Kartoffelstärke, Gelatine, Tragakanth, Methylcellulose und/oder Polyvinylpyrrolidon, und, wenn erwünscht, Sprengmittel, wie die obengenannten Stärken, ferner Carboxymethylstärke, quervernetzes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Als topisch anwendbare pharmazeutische Präparate kommen in erster Linie Cremes, Salben, Schäume, Tinkturen und Lösungen, in Frage, die von etwa 0,5 bis etwa 20 % des Wirkstoffs enthalten.

Cremes sind Oel-in-Wasser Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyethylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 %, Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoff, z.B. Paraffin, Vaseline und/oder flüssige Paraffine, ferner natürliches oder partialsynthetisches Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Cremes und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylensorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerigethanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Ethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die Dosierung des Wirkstoffs hängt von der Warmblüter-Spezies, dem Alter und dem individuellen Zustand sowie der Applikationsweise ab. Im Normalfall ist für einen etwa 75 kg schweren Warmblüter bei oraler Applikation eine ungefähre Tagesdosis von etwa 100 mg bis etwa 1000 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1:

15 g Benzo[b]thiophen-2(3H)-on (100 mM) werden in einem Sulfierkolben unter Stickstoffatmosphäre in 150 ml destilliertem Hexametapol gelöst. Die hellgelbe Lösung wird auf 5° abgekühlt. Anschliessend werden 8,9 g einer mit n-Hexan entölten Natriumhydrid-Suspension (55%ig) in Portionen unter Rühren zugegeben. Die gebildete Suspension wird bei 20° 15 Minuten gerührt, wobei eine Gasentwicklung zu beobachten ist. Anschliessend wird auf -10° abgekühlt und mit 17,65 g 2-(2-Thienyl)essigsäurechlorid versetzt, wobei die Innentemperatur auf unter 0° gehalten wird. Nach Entfernen der Kühlung wird 1 Stunde nachgerührt. Das Reaktionsgemisch wird zunächst auf 2 l Eiswasser und 60 ml 2 N Salzsäure gegossen und anschliessend mit 500 ml Toluol versetzt. Die abgetrennte organische Phase wird 2 mal mit Wasser und dann 3 mal mit je 200 ml 5%iger Sodalösung extrahiert. Die alkalische Phase wird filtriert und auf Eis und 2N Salzsäure gegossen, und die gebildeten Kristalle werden abfiltriert und getrocknet. Nach Chromatographie über Kieselgel und Umkristallisation aus Methanol erhält man das 2-Hydroxy-3-[2-(2-thienyl)-acetyl]-benzo[b]thiophen,

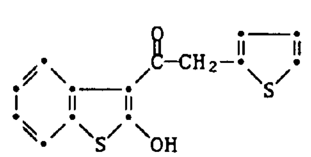

Smp. 99-101°, welches in 100 ml Methanol digeriert und mit 13,68 ml 30%iger Natriummethylatlösung versetzt wird. Nach Eindampfen zur Trockene wird zunächst mit 200 ml Ether und anschliessend mit Aceton versetzt. Das kristalline Natriumsalz schmilzt bei 201-204°.

Beispiel 2:

In einem Sulfierkolben werden unter Stickstoffatmosphäre 11 g 5-Fluor-benzo[b]thiophen-2(3H)-on in 110 ml destilliertem Hexametapol gelöst. Die Lösung wird auf 0° abgekühlt. Anschliessend werden 5,71 g einer mit n-Hexan entölten Natriumhydrid-Suspension (55%ig) in Portionen unter Rühren zugegeben, worauf eine Gasentwicklung einsetzt. Das Reaktionsgemisch wird bei 15° gehalten und gerührt, bis die Gasentwicklung beendet ist. Bei einer Temperatur zwischen -5° und 0° werden nun 11,55 g 2-(2-Thienyl)-essigsäurechlorid zugetropft. Es wird auf 50° Innentemperatur erwärmt, und die Gasentwicklung setzt wieder ein. Nach weiterem Rühren wird das Reaktionsgemisch auf 2 l Eiswasser und 50 ml 2N Salzsäure gegossen und mit 200 ml Toluol versetzt. Die organische Phase wird 2 mal mit Wasser und dann 3 mal mit je 150 ml 3%iger Sodalösung extrahiert. Die alkalische Lösung wird filtriert und auf Eiswasser mit 2N Salzsäure gegossen. Die gebildeten Kristalle werden abfiltriert, getrocknet und gelöst in 300 ml Toluol über Kieselgel chromatographiert. Der kristalline Rückstand, das 5-Fluor-2-hydroxy-3-[2-(2-thienyl)-acetyl]-benzo[b]thiophen (Smp. 134-136°), wird in 100 ml Methanol digeriert und mit 8,9 ml 30%iger Natriummethylatlösung versetzt. Die Lösung wird nun am Rotavap eingeengt, der Rückstand mit Ether digeriert und filtriert. Der Rückstand wird in 50 ml Aceton gelöst, auf 400 ml Ether gegossen, der kristalline Rückstand filtriert und im Vakuum getrocknet. Man erhält so das Natriumsalz des 5-Fluor-2-hydroxy-3-[2-(2-thienyl)-acetyl]-benzo[b]thiophen, Smp. 226-230°.

Beispiel 3:

In analoger Weise wie in Beispiel 1 oder 2 beschrieben kann man ausgehend von 4 g 6-Fluor-benzo[b]thiophen-2(3H)-on und 4,57 g 2-(2-Thienyl)-essigsäurechlorid das 6-Fluor-2-hydroxy-3-[2-(2-thienyl)acetyl]-benzo[b]thiophen herstellen. Das analog Beispiel 1 oder 2 gewonnene Natriumsalz schmilzt bei 264-268°.

Beispiel 4:

In analoger Weise wie in Beispiel 1 oder 2 beschrieben kann man ausgehend von 4 g 6-Fluor-benzo[b]thiophen-2(3H)-on und 4,31 g 2-(4-Fluorphenyl)-essigsäurechlorid das 6-Fluor-2-hydroxy-3-[2-(4-fluorphenyl)-acetyl]-benzo[b]thiophen herstellen, Smp. 106-108. Das analog Beispiel 1 oder 2 gewonnene Natriumsalz schmilzt bei 254-257°.

Beispiel 5:

In analoger Weise wie in Beispiel 1 oder 2 beschrieben kann man ausgehend von 4 g 6-Fluor-benzo[b]thiophen-2(3H)-on und 3,86 g 2-Phenylacetylchlorid das 6-Fluor-2-hydroxy-3-(2-phenylacetyl)-benzo[b]tiophen herstellen, Smp. 64-66°. Das analog Beispiel 1 oder 2 gewonnene Natriumsalz schmilzt bei 270-275°.

Beispiel 6:

In analoger Weise wie in Beispiel 1 oder 2 beschrieben kann man ausgehend von 5 g 5-Fluor-benzo[b]thiophen-2(3H)-on und 5,06 g 2-Phenylacetylchlorid das 5-Fluor-2-hydroxy-3-(2-phenylacetyl)-benzo[b]thiophen herstellen, Smp. 109-111°. Das analog Beispiel 1 oder 2 gewonnene Natriumsalz schmilzt bei 265-269°.

Beispiel 7:

In analoger Weise wie in Beispiel 1 oder 2 beschrieben kann man ausgehend von 10 g Benzo[b]thiophen-2(3H)-on und 12,63 g 2-(3-Fluorphenyl)-acetylchlorid das 2-Hydroxy-3-[2-(3-fluorphenyl)-acetyl]-benzo[b]thiophen herstellen, Smp. 88-90°. Das analog Beispiel 1 oder 2 gewonnene Natriumsalz schmilzt bei 213-216°.

Beispiel 8:

In analoger Weise wie in Beispiel 1 oder 2 beschrieben kann man ausgehend von 5 g 6-Fluor-benzo[b]thiophen-2(3H)-on und 7,37 g 2-(3-Trifluormethylphenyl)-acetylchlorid das 6-Fluor-2-hydroxy-3-[2-(3-trifluormethylphenyl)-acetyl]-benzo[b]thiophen herstellen, Smp. 77-79°. Das analog Beispiel 1 oder 2 gewonnene Natriumsalz schmilzt bei 200-203°.

Beispiel 9:

In analoger Weise wie in Beispiel 1 oder 2 beschrieben kann man ausgehend von 7,4 g Benzo[b]thiophen-2(3H)-on und 10,01 g 2-(4-Methoxyphenyl)-acetylchlorid das2-Hydroxy-3-[2-(4-methoxyphenyl)-acetyl]-benzo[b]thiophen, Smp. 122-123°, herstellen. Das analog Beispiel 1 oder 2 gewonnene Natriumsalz schmilzt bei 155-157°.

Beispiel 10:

In analoger Weise wie in Beispiel 1 oder 2 beschrieben kann man ausgehend von 7,18 g Benzo[b]thiophen-2(3H)-on und 8,14 g Phenylacetylchlorid das 2-Hydroxy-3-(2-phenylacetyl)-benzo[b]thiophen, Smp. 101-103°, herstellen. Das analog Beispiel 1 oder 2 gewonnene Natriumsalz schmilzt bei 227-230°.

Beispiel 11:

In analoger Weise wie in Beispiel 1 oder 2 beschrieben kann man ausgehend von 7,62 g Benzo[b]thiophen-2(3H)-on und 9,63 g 2-(4-Fluorphenyl)-acetylchlorid das 2-Hydroxy-3-[2-(4-fluorphenyl)-acetyl]-benzo[b]thiophen, Smp. 105-106°, herstellen. Das analog Beispiel 1 oder 2 gewonnene Natriumsalz schmilzt bei 238-241°.

Beispiel 12:

In analoger Weise wie in Beispiel 1 oder 2 beschrieben kann man ausgehend von 7,13 g Benzo[b]thiophen-2(3H)-on und 9,8 g 2-Phenyl-propionylchlorid das 2-Hydroxy-3-(2-phenyl-propionyl)-benzo[b]thiophen, Smp. 82-83°, herstellen. Das analog Beispiel 1 oder 2 gewonnene Natriumsalz schmilzt bei 153-156°.

Beispiel 13:

In analoger Weise wie in Beispiel 1 oder 2 beschrieben kann man ausgehend von 7,4 g 6-Chlor-benzo[b]thiophen-2(3H)-on und 7,08 g 2-Thiophenessigsäurechlorid das 6-Chlor-2-hydroxy-3-[2-(2-thienyl)-acetyl]-benzo[b]thiophen, Smp. 77-78°, herstellen. Das analog Beispiel 1 oder 2 gewonnene Natriumsalz schmilzt bei 211-214°.

Beispiel 14:

In analoger Weise wie in Beispiel 1 oder 2 beschrieben kann man herstellen:
2-Hydroxy-3-[2-(4-chlorphenyl)-acetyl]-benzo[b]thiophen Smp. 112-114°;
Na-Salz: Smp. 252-255°;
5-Fluor-2-hydroxy-3-[2-(2-pyridyl)-acetyl]-benzo[b]thiophen;
5-Fluor-2-hydroxy-3-[2-(3-pyrrolyl)-acetyl]-benzo[b]thiophen;
2-Hydroxy-3-[2-(2-thienyl)-acetyl]-benzo[b]thiophen-1-oxid;
2-Hydroxy-3-[2-(2-thienyl)-acetyl]-benzo[b]thiophen-1, 1-dioxid;
2-Hydroxy-3-(2-phenylacetyl)-benzo[b]thiophen-1-oxid;
2-Hydroxy-3-(2-phenylacetyl)-benzo[b]thiophen-1, 1-dioxid.

Beispiel 15:

8.41 g Benzo[b]thiophen-2(3H)-on (100 mM) werden in einem Sulfierkolben unter Stickstoffatmosphäre in 150 ml destilliertem Hexametapol gelöst. Die hellgelbe Lösung wird auf 5° abgekühlt. Anschliessend werden 8,9 g einer mit n-Hexan entölten Natriumhydrid-Suspension (55%ig) in Portionen unter Rühren zugegeben. Die gebildete Suspension wird bei 20° 15 Minuten gerührt, wobei eine Gasentwicklung zu beobachten ist. Anschliessend werden bei 30° bis 40° 11,66 g 2-(4-Chlorphenyl)-essigsäurechlorid zugetropft. Anschliessend wird 30 Minuten bei 50° nachgerührt. Das Reaktionsgemisch wird zunächst auf 2 l Eiswasser und 60 ml 2 N Salzsäure gegossen und anschliessend mit 500 ml Toluol versetzt. Die abgetrennte organische Phase wird 2 mal mit Wasser und dann 3 mal mit je 200 ml 5%iger Sodalösung extrahiert. Die alkalische Phase wird filtriert und auf Eis und 2N Salzsäure gegossen, und das gebildete Oel mit n-Hexan versetzt und gerührt. Die gebildeten Kristalle werden abfiltriert, mit n-Hexan/Ether (1:1) gewaschen und im Vakuum getrocknet. Man erhält so 2-Hydroxy-3-[2-(4-chlorphenyl)-acetyl]-benzo[b]thiophen, Smp. 112-114°, welches in 50 ml Methanol digeriert und mit 5,44 ml 30%iger Natriummethylatlösung bei pH 8-9 versetzt wird. Nach Eindampfen zur Trockene wird zunächst mit Ether versetzt. Das kristalline Natriumsalz schmilzt bei 252-255°.

Beispiel 16:

In einem Sulfierkolben werden unter Stickstoffatmosphäre 3 g 5-Fluor-benzo[b]thiophen-2(3H)-on in 30 ml destilliertem Hexametapol gelöst. Die Lösung wird auf 0° abgekühlt. Anschliessend werden 1,63 g einer mit

n-Hexan entölten Natriumhydrid-Suspension (55%ig) in Portionen unter Rühren zugegeben, worauf eine Gasentwicklung einsetzt. Das Reaktionsgemisch wird 30 Minuten bei Raumtemperatur gehalten und gerührt, bis die Gasentwicklung beendet ist. Bei einer Innentemperatur zwischen 35° und 45° werden nun 3,14 g 2-(3-Thienyl)-essigsäurechlorid zugetropft. Es wird auf 50° Innentemperatur erwärmt, und die Gasentwicklung setzt wieder ein. Nach weiterem Rühren wird das Reaktionsgemisch auf 2 l Eiswasser und 50 ml 2N Salzsäure gegossen und mit 200 ml Toluol versetzt. Die organische Phase wird 2 mal mit Wasser und dann 3 mal mit je 150 ml 5%iger Sodalösung extrahiert. Die alkalische Lösung wird filtriert und auf Eiswasser mit 2N Salzsäure gegossen. Die gebildeten Kristalle werden abfiltriert, getrocknet und 3 mal mit n-Hexan gewaschen. Der kristalline Rückstand, das 5-Fluor-2-hydroxy-3-[2-(3-thienyl)-acetyl]-benzo[b]thiophen (Smp. 122-124°), wird in 50 ml Methanol digeriert und mit 2,91 ml 30%iger Natriummethylatlösung versetzt. Die Lösung wird nun am Rotavap eingeengt und im Vakuum getrocknet. Der Rückstand wird mit 30 ml Ether digeriert und gerührt. Es bilden sich Kristalle, die abfiltriert, mit Ether gewaschen und filtriert und im Vakuum getrocknet werden. Man erhält so das Natriumsalz des 5-Fluor-2-hydroxy-3-[2-(3-thienyl)-acetyl]-benzo[b]thiophen, Smp. 278-281°.

Beispiel 17:

4.83 g Benzo[b]thiophen-2(3H)-on werden in einem Sulfierkolben unter Stickstoffatmosphäre in 50 ml destilliertem Hexametapol gelöst. Die hellgelbe Lösung wird auf 5° abgekühlt. Anschliessend werden 3,09 g einer mit n-Hexan entölten Natriumhydrid-Suspension (55%ig) in Portionen unter Rühren zugegeben. Die gebildete Suspension wird bei 20° 15 Minuten gerührt, wobei eine Gasentwicklung zu beobachten ist. Anschliessend wird mit 14,4 g 2-(1-Methyl-pyrrol-2-yl)-essigsäure-isobutylkohlensäure-anhydrid versetzt, wobei die Innentemperatur auf 31° ansteigt. Es wird nun mittels eines Wasserbads auf 60° Innentemperatur erwärmt. Das Reaktionsgemisch wird zunächst auf 2 l Eiswasser und 60 ml 2 N Salzsäure gegossen, wobei ein Oel ausfällt. Es wird 2 mal mit Essigester extrahiert, mit Wasser gewaschen und über $MgSO_4$ getrocknet. Das gebildete Oel wird im Ether digeriert, mit Aktivkohle versetzt und über einen Hyflofilter abgesaugt. Nach Eindampfen der Lösung wird im Vakuum getrocknet. Der Rückstand wird dann in Toluol gelöst und auf eine Kieselgelsäure (20 g; mit Toluol konditioniert) bei 0,4 bar aufgepresst. Das erhaltene Oel wird in Ether und n-Hexan digeriert. Man erhält man so 2-Hydroxy-3-[2-(1-methylpyrrol-2-yl)-acetyl]-benzo[b]thiophen, Smp. 94-96°, welches in Methanol digeriert und mit 1,2 ml 30%iger Natriummethylatlösung versetzt wird. Nach Eindampfen zur Trockene wird zunächst in Ether digeriert und gerührt. Das gebildete kristalline Natriumsalz schmilzt bei 249-253°.

Beispiel 18:

In einem Sulfierkolben werden unter Stickstoffatmosphäre 6,44 g 5-Chlor-benzo[blthiophen-2(3H)-on in 50 ml destilliertem Hexametapol gelöst. Die Lösung wird auf 0° abgekühlt. Anschliessend werden 3,19 g einer mit n-Hexan entölten Natriumhydrid-Suspension (55%ig) in Portionen unter Rühren zugegeben, worauf eine Gasentwicklung einsetzt. Das Reaktionsgemisch wird bei 15° gehalten und gerührt, bis die Gasentwicklung beendet ist. Bei einer Temperatur zwischen 30° und 35° Innentemperatur werden nun 6,16 g 2-(2-Thienyl)-essigsäurechlorid zugetropft. Es wird auf 50° Innentemperatur erwärmt und 15 Minuten gerührt. Das Reaktionsgemisch wird auf 2 l Eiswasser und 50 ml 2N Salzsäure gegossen und mit 200 ml Toluol versetzt. Die organische Phase wird 2 mal mit Wasser und dann 3 mal mit je 150 ml 3%iger Sodalösung extrahiert. Die alkalische Lösung wird filtriert und auf Eiswasser mit 2N Salzsäure gegossen. Die gebildeten Kristalle werden abfiltriert, in Methylenchlorid gelöst, über $MgSO_4$ getrocknet. Das erhaltene Oel wird mit 100 ml n-Hexan aufgekocht. Die gebildeten Kristalle werden abgefiltert und getrocknet. Man erhält so das 5-Chlor-2-hydroxy-3-[2-(2-thienyl)-acetyl]-benzo[b]thiophen (Smp. 129-130°). Dieses wird in 50 ml Methanol digeriert und mit 4,38 ml 30%iger Natriummethylatlösung versetzt. Die Lösung wird nun am Rotavap eingeengt, der Rückstand mit Ether digeriert, gerührt und filtriert. Der Rückstand wird mit Ether gewaschen, der kristalline Rückstand filtriert und im Vakuum getrocknet. Man erhält so das Natriumsalz des 5-Chlor-2-hydroxy-3-[2-(2-thienyl)-acetyl]-benzo[b]thiophen, Smp. 258-261°.

Beispiel 19:

3 g 5-Nitrobenzo[b]thiophen-2(3H)-on werden in einem Sulfierkolben unter Stickstoffatmosphäre in 50 ml destilliertem Hexametapol gelöst. Die hellgelbe Lösung wird auf 5° abgekühlt. Anschliessend werden 1,44 g einer mit n-Hexan entölten Natriumhydrid-Suspension (55%ig) in Portionen unter Rühren zugegeben. Die gebildete Suspension wird bei 20° 15 Minuten gerührt, wobei eine Gasentwicklung zu beobachten ist. Anschliessend wird bei 30° bis 40° mit 2,71 g 2-(2-Thienyl)-essigsäurechlorid versetzt und bei 50° 15 Minuten gerührt. Das Reaktionsgemisch wird zunächst auf 2 l Eiswasser und 60 ml 2 N Salzsäure gegossen und anschliessend

mit 500 ml Toluol versetzt. Die abgetrennte organische Phase wird 2 mal mit Wasser und dann 3 mal mit je 200 ml 5%iger Soda lösung extrahiert. Die alkalische Phase wird filtriert und auf Eis und 2N Salzsäure gegossen, und die gebildeten Kristalle werden abfiltriert und getrocknet. Dann wird 2 mal mit Ether digeriert, filtriert und im Vakuum getrocknet. Man erhält so das 2-Hydroxy-5-nitro-3-[2-(2-thienyl)-acetyl]-benzo[b]thiophen, Smp. 128-130°.

Beispiel 20:

In analoger Weise, beispielsweise wie in einem der vorstehenden Beispiele beschrieben, kann man ausgehend von Benzo[b]thiophen-2(3H)-on und 3-(2-Thienyl)-propionsäurechlorid das 2-Hydroxy-3-[3-(2-thienyl)-propionyl]-benzo[b]thiophen herstellen. Das analog Beispiel 1 oder 2 gewonnene Natriumsalz schmilzt bei 259-262°.

Beispiel 21:

In analoger Weise, beispielsweise wie in Beispiel 1 oder 2 beschrieben, kann man ausgehend von 2.03 g Benzo[b]thiophen-2(3H)-on und 3.1 g 5-Brom-2-thiophenessigsäurechlorid das 2-Hydroxy-3-[2(5-brom-2-thienyl)-acetyl]-benzo[b]thiophen, Smp. 87-88°C, herstellen. Das analog Beispiel 1 oder 2 gewonnene Natriumsalz schmilzt bei 214-217°C.

Beispiel 22:

In analoger Weise, beispielsweise wie in Beispiel 1 oder 2 beschrieben, kann man herstellen:
2-Hydroxy-3-[2-(3-methyl-2-thienyl)-acetyl]-benzo[b]thiophen.
Smp. 82-84°C. Das analog Beispiel 1 oder 2 gewonnene Natriumsalz schmilzt bei 237-240°C.

Beispiel 23:

In analoger Weise, z.B. wie in einem der vorstehenden Beispiele beschrieben, kann man herstellen:
2-Hydroxy-3-[ 2-(2-pyridyl)-acetyl]-benzo[b]thiophen;
5-Fluor-2-hydroxy-3-[2-(2-thiazolyl)-acetyl]-benzo[b]thiophen;
5-Fluor-2-hydroxy-3-[2-(3-methyl-2-pyridyl)-acetyl]-benzo[b]thiophen;
2-Hydroxy-3-[2-(4-chlor-imidazol-1-yl)-acetyl]-benzo[b]thiophen;
2-Hydroxy-3-[2-(3-brom-2-pyridyl)-acetyl]-benzo[b]thiophen;
5-Fluor-2-hydroxy-3-[2-(imidazol-2-yl)-acetyl]-benzo[b]thiophen.

Beispiel 24:

Tabletten enthaltend 25 mg Wirkstoff, z.B. das Natriumsalz des 5-Fluor-2-hydroxy-3-[2-(2-thienyl)-acetyl]-benzo[b]thiophens, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung

Sämtliche festen Ingredienzen werden zunächst durch ein Sieb von 0,6 mm Maschenweite getrieben. Dann wird die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben, mit der Pulvermischung zu einer knetbaren Masse verarbeitet und das erhaltene Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 25:

In analoger Weise wie in Beispiel 24 beschrieben, können auch Tabletten, enthaltend jeweils 25 mg einer anderen der in den Beispielen 1 bis 23 genannten Verbindungen, hergestellt werden.

## Patentansprüche

### Patentansprüche für folgende Vertragsstaaten : AT, BE, CH, LI, DE, FR, GB, IT, LU, NL, SE

**1.** Verbindungen der Formel

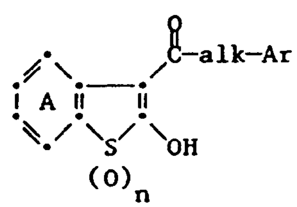

(I) bzw.

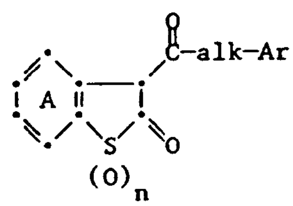

(Ia)

und ihre Salze, worin der Index n 0, 1 oder 2 bedeutet, alk Niederalkylen bedeutet, Ar Phenyl, Naphthyl oder einen monocyclischen, fünf- oder sechsgliedrigen Heteroarylrest bedeutet, wobei der aromatische Rest unsubstituiert oder ein- oder mehrfach durch Niederalkyl, Halogenniederalkyl, Halogen und/oder Nitro substituiert ist, und der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist, wobei unter "niederen" Reaten solche zu verstehen sind, die bis und mit sieben C-Atome aufweisen.

**2.** Verbindungen gemäss Anspruch 1 der Formeln I und Ia sowie ihre Salze, worin alk für -CH(R)- steht und R Wasserstoff oder Niederalkyl bedeutet.

**3.** Verbindungen gemäss Anspruch 1 der Formeln I und Ia sowie ihre Salze, worin der Index n 0, 1 oder 2 bedeutet, alk für Niederalkylen, insbesondere für -CH(R)- und R für Wasserstoff oder Niederalkyl steht, Ar jeweils unsubstituiertes oder ein- oder mehrfach durch Niederalkyl, Halogenniederalkyl, Halogen und/oder Nitro substituiertes Phenyl, Naphthyl, Pyrrolyl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, wobei in entsprechenden Heteroarylresten, die die -NH-Gruppe aufweisen, das betreffende Wasserstoffatom durch Niederalkyl substituiert sein kann, und der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist.

**4.** Verbindungen gemäss Anspruch 2 der Formeln I und Ia sowie ihre Salze, worin der Index n 0, 1 oder 2 bedeutet, Ar jeweils unsubstituiertes oder ein- oder mehrfach durch Niederalkyl, Halogenniederalkyl, Halogen und/oder Nitro substituiertes Phenyl, Naphthyl, Pyrrolyl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist, und alk für -CH(R)-steht, wobei R Wasserstoff oder Niederalkyl bedeutet.

5. Verbindungen gemäss Anspruch 1 der Formeln I und Ia sowie ihre Salze, worin der Index n für 0, ferner 1 oder 2 steht, alk für Niederalkylen, insbesondere für -CH(R)- und R für Wasserstoff oder Niederalkyl steht, Ar unsubstituiertes oder ein- oder mehrfach durch Niederalkyl, Niederalkoxy, Halogenniederalkyl, insbesondere Trifluoromethyl, oder Halogen substituiertes Phenyl, Pyrrolyl, 1-Niederalkyl-pyrrolyl, Thienyl, Thiazolyl oder Pyridyl bedeutet und der Ring A unsubstituiert oder ein- oder mehrfach durch Niederalkyl, Niederalkoxy, Halogenniederalkyl, insbesondere Trifluormethyl, Halogen oder Nitro substituiert ist.

6. Verbindungen gemäss Anspruch 2 der Formeln I und Ia sowie ihre Salze, worin der Index n für 0, 1 oder 2 steht, alk für -CH(R)- steht und R Wasserstoff oder Niederalkyl bedeutet, Ar unsubstituiertes oder ein- oder mehrfach durch Halogenniederalkyl, insbesondere Trifluormethyl, oder Halogen substituiertes Phenyl, Pyrrolyl, Thienyl oder Pyridyl bedeutet und der Ring A unsubstituiert oder ein- oder mehrfach durch Niederalkyl, Niederalkoxy, Halogenniederalkyl, insbesondere Trifluormethyl, Halogen oder Nitro substituiert ist.

7. Verbindungen gemäss Anspruch 1 der Formeln I und Ia sowie ihre Salze, worin der Index n 0 bedeutet, alk für -CH(R)- steht und R Niederalkyl, insbesondere mit bis und mit 4 C-Atomen, wie Methyl, bedeutet, Ar unsubstituiertes oder durch Niederalkoxy, insbesondere mit bis und mit 4 C-Atomen, wie Methoxy, Halogen, insbesondere mit Atomnummer bis und mit 35, wie Fluor oder Chlor, oder Trifluormethyl, jeweils insbesondere in Position 3 oder 4 lokalisiert, substituiertes Phenyl, Thiazolyl, wie 2-Thiazolyl, oder Pyridyl, wie 2-Pyridyl, oder unsubstituiertes 1-Niederalkyl-pyrrolyl, insbesondere mit bis und mit 4 C-Atomen im Niederalkylteil, wie 1-Methyl-pyrrol-2-yl, oder unsubstituiertes oder durch Niederalkyl, insbesondere mit bis und mit 4 C-Atomen, wie Methyl, oder Halogen, insbesondere mit Atomnummer bis und mit 35, wie Brom, substituiertes Thienyl, wie 2- oder 3-Thienyl, bedeutet und der Ring A unsubstituiert oder durch Halogen, insbesondere mit Atomnummer bis und mit 35, wie Fluor oder Chlor, oder Nitro, insbesondere in Position 5 oder 6 des Ringsystems, substituiert ist.

8. Verbindungen gemäss Anspruch 2 der Formel I und Ia sowie ihre Salze, worin der Index n 0, ferner 1 oder 2 bedeutet, alk für -CH(R)-steht und R Wasserstoff und Niederalkyl, insbesondere mit bis und mit 4 C-Atomen, bedeutet, Ar unsubstituiertes oder durch Halogenniederalkyl, insbesondere mit bis und mit 2 C-Atomen, wie Trifluormethyl, oder Halogen, insbesondere mit Atomnummer bis und mit 35, wie Fluor oder Chlor, substituiertes Phenyl, oder unsubstituiertes Pyrrolyl, Thienyl oder Pyridyl bedeutet und der Ring A unsubstituiert oder durch Halogen, insbesondere mit Atomnummer bis und mit 35, wie Fluor oder Chlor, oder Nitro monosubstituiert ist.

9. Verbindungen gemäss Anspruch 2 der Formeln I und Ia sowie ihre Salze, worin der Index n 0 ist, alk für -CH(R)- steht und R Wasserstoff ist, Ar unsubstituiertes oder durch Trifluormethyl oder Halogen mit Atomnummer bis und mit 35, wie Fluor, insbesondere in Position 3 oder 4, substituiertes Phenyl oder unsubstituiertes Thienyl, wie 2-Thienyl, bedeutet und der Ring A unsubstituiert oder durch Halogen mit Atomnummer bis und mit 35, wie Fluor, insbesondere in Position 5 oder 6 des Ringsystems, substituiert ist.

10. Verbindungen gemäss Anspruch 1 der Formeln I und Ia sowie ihre Salze, worin der Index n 0 ist, alk für -CH(R)- und R für Wasserstoff steht, Ar Phenyl oder 2- oder 3-Thienyl bedeutet und der Ring A unsubstituiert oder in Position 5 oder 6 des Ringsystems durch Fluor oder Chlor substituiert ist.

11. Verbindungen gemäss Anspruch 2 der Formeln I und Ia sowie ihre Salze, worin der Index n 0 ist, alk für -CH(R)-steht und R Wasserstoff ist, Ar Thienyl, wie 2-Thienyl, bedeutet und der Ring A unsubstituiert oder durch Fluor, insbesondere in Position 5 oder 6 des Ringsystems, substituiert ist.

12. 2-Hydroxy-3-[2-(2-thienyl)-acetyl]-benzo[b]thiophen oder ein Salz davon.

13. 5-Fluor-2-hydroxy-3-[2-(2-thienyl)-acetyl]-benzo[b]thiophen oder ein Salz davon.

14. 6-Fluor-2-hydroxy-3-[2-(2-thienyl)-acetyl]-benzo[b]thiophen oder ein Salz davon.

15. 5-Fluor-2-hydroxy-3-(2-phenylacetyl)-benzo[b]thiophen oder ein Salz davon.

16. 6-Chlor-2-hydroxy-3-[2-(2-thienyl)-acetyl]-benzo[b]thiophen oder ein Salz davon.

17. 5-Chlor-2-hydroxy-3-[2-(2-thienyl)-acetyl]-benzo[b]thiophen oder ein Salz davon.

**18.** 5-fluor-2-hydroxy-3-[2-(3-thienyl)-acetyl]-benzo[b]thiophen oder ein Salz davon.

**19.** 2-Hydroxy-3-[3-(2-thienyl)-propionyl]-benzo[b]thiophen oder ein Salz davon.

**20.** Verbindung gemäss einem der Ansprüche 1-19 zur Anwendung in einem Verfahren zur Behandlung des menschlichen oder tierischen Körpers.

**21.** Pharmazeutische Präparate enthaltend eine Verbindung gemäss einem der Ansprüche 1-20 oder ein pharmazeutisch verwendbares Salz davon.

**22.** Verwendung von Verbindungen gemäss einem der Ansprüche 1-20 zur Herstellung von Analgetika.

**23.** Verfahren zur Herstellung von Verbindungen gemäss einem der Ansprüche 1-19, dadurch gekennzeichnet, dass man

a) eine Verbindung der Formel

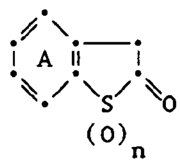

(IIa),

ein Tautomeres oder Salz davon mit einer Verbindung der Formel $X_1$-CO-alk-Ar (IIb), worin $X_1$ reaktionsfähiges verestertes Hydroxy bedeutet, umsetzt oder

b) eine Verbindung der Formel

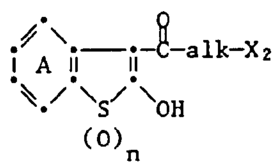

(IIIa),

ein Tautomeres oder Salz davon, worin $X_2$ reaktionsfähiges verestertes Hydroxy bedeutet, mit einer Verbindung der Formel Ar-H (IIIb) umsetzt

und gewünschtenfalls eine verfahrensgemäss oder auf andere Weise erhältliche Verbindung in eine andere Verbindung der Formel I bzw. Ia überführt, ein verfahrensgemäss erhältliches Isomerengemisch in die Komponenten auftrennt, eine verfahrensgemäss erhältliche freie Verbindung der Formel I bzw. Ia in ein Salz überführt und/oder ein verfahrensmäss erhältliches Salz in die freie Verbindung der Formel I bzw. Ia oder in ein anderes Salz überführt.

**Patentansprüche für folgende Vertragsstaaten : ES, GR**

**1.** Verfahren zur Herstellung von Verbindungen der Formel

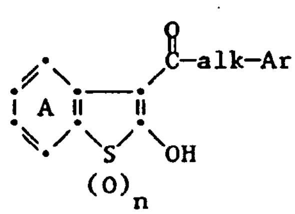

(I) bzw.

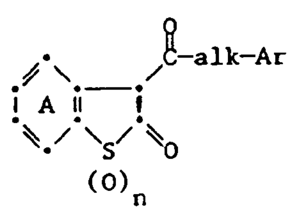

(Ia)

und ihrer Salze, worin der Index n 0, 1 oder 2 bedeutet, alk Niederalkylen bedeutet, Ar Phenyl, Naphthyl oder einen monocyclischen, fünf- oder sechsgliedrigen Heteroarylrest bedeutet, wobei der aromatische Rest unsubstituiert oder ein- oder mehrfach durch Niederalkyl, Halogenniederalkyl, Halogen und/oder Nitro substituiert ist, und der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist, wobei unter "niederen" Reaten solche zu verstehen sind, die bis und sieben C-Atome aufweisen, dadurch gekennzeichnet, dass man

a) eine Verbindung der Formel

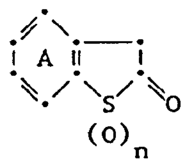

(IIa),

ein Tautomeres oder Salz davon mit einer Verbindung der Formel $X_1$-CO-alk-Ar (IIb), worin $X_1$ reaktionsfähiges verestertes Hydroxy bedeutet, umsetzt oder

b) eine Verbindung der Formel

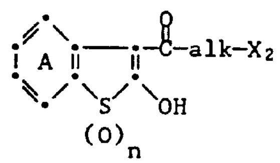

(IIIa),

ein Tautomeres oder Salz davon, worin $X_2$ reaktionsfähiges verestertes Hydroxy bedeutet, mit einer Verbindung der Formel Ar-H (IIIb) umsetzt

und gewünschtenfalls eine verfahrensgemäss oder auf andere Weise erhältliche Verbindung in eine andere Verbindung der Formel I bzw. Ia überführt, ein verfahrensgemäss erhältliches Isomerengemisch in die Komponenten auftrennt, eine verfahrensgemäss erhältliche freie Verbindung der Formel I bzw. Ia in ein Salz überführt und/oder ein verfahrensmäss erhältliches Salz in die freie Verbindung der Formel I bzw Ia oder in ein anderes Salz überführt.

2. Verfahren gemäss Anspruch 1 zur Herstellung von Verbindungen der Formel I und Ia und ihrer Salze, worin alk für -CH(R)- steht und R Wasserstoff oder Niederalkyl bedeutet.

3. Verfahren gemäss Anspruch 1 zur Herstellung von Verbindungen der Formeln I und Ia sowie ihrer Salze, worin der Index n 0, 1 oder 2 bedeutet, alk für Niederalkylen, insbesondere für -CH(R)- und R für Wasserstoff oder Niederalkyl steht, Ar jeweils unsubstituiertes oder ein- oder mehrfach durch Niederalkyl, Halogenniederalkyl, Halogen und/oder Nitro substituiertes Phenyl, Naphthyl, Pyrrolyl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, wobei in entsprechenden Heteroarylresten, die die -NH-Gruppe aufweisen, das betreffende Wasserstoffatom durch Niederalkyl substituiert sein kann, und der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist.

4. Verfahren gemäss Anspruch 2 zur Herstellung von Verbindungen der Formeln I und Ia sowie ihrer Salze, worin der Index n 0, 1 oder 2 bedeutet, Ar jeweils unsubstituiertes oder ein- oder mehrfach durch Niederalkyl, Halogenniederalkyl, Halogen und/oder Nitro substituiertes Phenyl, Naphthyl, Pyrrolyl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl oder Triazinyl bedeutet, der Ring A unsubstituiert oder ein- oder mehrfach durch Niederalkyl, Niederalkoxy, Niederalkylthio, Niederalkansulfinyl, Niederalkansulfonyl, Halogenniederalkyl, Halogen und/oder Nitro substituiert ist, und alk für -CH(R)-steht, wobei R Wasserstoff oder Niederalkyl bedeutet.

5. Verfahren gemäss Anspruch 1 zur Herstellung von Verbindungen der Formeln I und Ia sowie ihrer Salze,

worin der Index n für 0, ferner 1 oder 2 steht, alk für Niederalkylen, insbesondere für -CH(R)- und R für Wasserstoff oder Niederalkyl steht, Ar unsubstituiertes oder ein- oder mehrfach durch Niederalkyl, Niederalkoxy, Halogenniederalkyl, insbesondere Trifluoromethyl, oder Halogen substituiertes Phenyl, Pyrrolyl, 1-Niederalkyl-pyrrolyl, Thienyl, Thiazolyl oder Pyridyl bedeutet und der Ring A unsubstituiert oder ein- oder mehrfach durch Niederalkyl, Niederalkoxy, Halogenniederalkyl, insbesondere Trifluormethyl, Halogen oder Nitro substituiert ist.

6. Verfahren gemäss Anspruch 2 zur Herstellung von Verbindungen der Formeln I und Ia sowie ihrer Salze, worin der Index n für 0, 1 oder 2 steht, alk für -CH(R)- steht und R Wasserstoff oder Niederalkyl bedeutet, Ar unsubstituiertes oder ein- oder mehrfach durch Halogenniederalkyl, insbesondere Trifluormethyl, oder Halogen substituiertes Phenyl, Pyrrolyl, Thienyl oder Pyridyl bedeutet und der Ring A unsubstituiert oder ein- oder mehrfach durch Niederalkyl, Niederalkoxy, Halogenniederalkyl, insbesondere Trifluormethyl, Halogen oder Nitro substituiert ist.

7. Verfahren gemäss Anspruch 1 zur Herstellung von Verbindungen der Formeln I und Ia sowie ihrer Salze, worin der Index n 0 bedeutet, alk für -CH(R)- steht und R Niederalkyl, insbesondere mit bis und mit 4 C-Atomen, wie Methyl, bedeutet, Ar unsubstituiertes oder durch Niederalkoxy, insbesondere mit bis und mit 4 C-Atomen, wie Methoxy, Halogen, insbesondere mit Atomnummer bis und mit 35, wie Fluor oder Chlor, oder Trifluormethyl, jeweils insbesondere in Position 3 oder 4 lokalisiert, substituiertes Phenyl, Thiazolyl, wie 2-Thiazolyl, oder Pyridyl, wie 2-Pyridyl, oder unsubstituiertes 1-Niederalkyl-pyrrolyl, insbesondere mit bis und mit 4 C-Atomen im Niederalkylteil, wie 1-Methyl-pyrrol-2-yl, oder unsubstituiertes oder durch Niederalkyl, insbesondere mit bis und mit 4 C-Atomen, wie Methyl, oder Halogen, insbesondere mit Atomnummer bis und mit 35, wie Brom, substituiertes Thienyl, wie 2- oder 3-Thienyl, bedeutet und der Ring A unsubstituiert oder durch Halogen, insbesondere mit Atomnummer bis und mit 35, wie Fluor oder Chlor, oder Nitro, insbesondere in Position 5 oder 6 des Ringsystems, substituiert ist.

8. Verfahren gemäss Anspruch 2 zur Herstellung von Verbindungen der Formel I und Ia sowie ihrer Salze, worin der Index n 0, ferner 1 oder 2 bedeutet, alk für -CH(R)-steht und R Wasserstoff und Niederalkyl, insbesondere mit bis und mit 4 C-Atomen, bedeutet, Ar unsubstituiertes oder durch Halogenniederalkyl, insbesondere mit bis und mit 2 C-Atomen, wie Trifluormethyl, oder Halogen, insbesondere mit Atomnummer bis und mit 35, wie Fluor oder Chlor, substituiertes Phenyl, oder unsubstituiertes Pyrrolyl, Thienyl oder Pyridyl bedeutet und der Ring A unsubstituiert oder durch Halogen, insbesondere mit Atomnummer bis und mit 35, wie Fluor oder Chlor, oder Nitro monosubstituiert ist.

9. Verfahren gemäss Anspruch 2 zur Herstellung von Verbindungen der Formeln I und Ia sowie ihrer Salze, worin der Index n 0 ist, alk für -CH(R)- steht und R Wasserstoff ist, Ar unsubstituiertes oder durch Trifluormethyl oder Halogen mit Atomnummer bis und mit 35, wie Fluor, insbesondere in Position 3 oder 4, substituiertes Phenyl oder unsubstituiertes Thienyl, wie 2-Thienyl, bedeutet und der Ring A unsubstituiert oder durch Halogen mit Atomnummer bis und mit 35, wie Fluor, insbesondere in Position 5 oder 6 des Ringsystems, substituiert ist.

10. Verfahren gemäss Anspruch 1 zur Herstellung von Verbindungen der Formeln I und Ia sowie ihrer Salze, worin der Index n 0 ist, alk für -CH(R)- und R für Wasserstoff steht, Ar Phenyl oder 2- oder 3-Thienyl bedeutet und der Ring A unsubstituiert oder in Position 5 oder 6 des Ringsystems durch Fluor oder Chlor substituiert ist.

11. Verfahren gemäss Anspruch 2 zur Herstellung von Verbindungen der Formeln I und Ia sowie ihrer Salze, worin der Index n 0 ist, alk für -CH(R)-steht und R Wasserstoff ist, Ar Thienyl, wie 2-Thienyl, bedeutet und der Ring A unsubstituiert oder durch Fluor, insbesondere in Position 5 oder 6 des Ringsystems, substituiert ist.

12. Verfahren gemäss Anspruch 2 zur Herstellung von Verbindungen 2-Hydroxy-3-[2-(2-thienyl)-acetyl]-benzo[b]thiophen oder eines Salzes davon.

13. Verfahren gemäss Anspruch 2 zur Herstellung von Verbindungen 5-Fluor-2-hydroxy-3-[2-(2-thienyl)-acetyl]-benzo[b]thiophen oder eines Salzes davon.

14. Verfahren gemäss Anspruch 2 zur Herstellung von Verbindungen 6-Fluor-2-hydroxy-3-[2-(2-thienyl)-acetyl]-benzo[b]thiophen oder eines Saltes davon.

15. Verfahren gemäss Anspruch 2 zur Herstellung von Verbindungen 5-Fluor-2-hydroxy-3-(2-phenylacetyl)-benzo[b]thiophen oder eines Salzes davon.

16. Verfahren gemäss Anspruch 2 zur Herstellung von Verbindungen 6-Chlor-2-hydroxy-3-[2-(2-thienyl)-acetyl]-benzo[b]thiophen oder eines Salzes davon.

17. Verfahren gemäss Anspruch 2 zur Herstellung von Verbindungen 5-Chlor-2-hydroxy-3-[2-(2-thienyl)-acetyl]-benzo[b]thiophen oder eines Salzes davon.

18. Verfahren gemäss Anspruch 1 zur Herstellung von Verbindungen 5-Fluor-2-hydroxy-3-[2-(3-thienyl)-acetyl]-benzo[b]thiophen oder eines Salzes davon.

19. Verfahren gemäss Anspruch 1 zur Herstellung von Verbindungen 2-Hydroxy-3-[3-(2-thienyl)-propionyl]-benzo[b]thiophen oder eines Salzes davon.

20. Verwendung von Verbindungen gemäss einem der Ansprüche 1-19 zur Herstellung von Analgetika.

21. Verfahren zur Herstellung von pharmazeutischen Präparaten, dadurch gekennzeichnet dass man eine Verbindung gemäss einem der Ansprüche 1-19 oder ein pharmazeutisch verwendbares Salz davon, gegebenenfalls unter Beimischung von üblichen Hilfs- und Zusatzstoffen, zu pharmazeutischen Präparaten verarbeitet.

## Claims

### Claims for the folllowing Contracting States : AT, BE, CH, LI, DE, FR, GB, IT, LU, NL, SE

1. A compound of formula

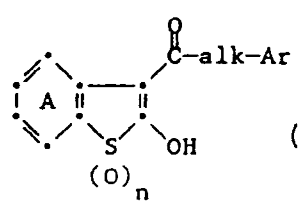 (I) or 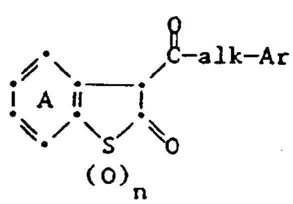 (Ia),

or a salt thereof, in which the index n is 0, 1 or 2, alk is lower alkylene, Ar is phenyl, naphthyl or a monocyclic, five- or six-membered heteroaryl radical, wherein the aromatic radical is unsubstituted or is mono- or poly-substituted by lower alkyl, halo-lower alkyl, halogen and/or by nitro, and the ring A is unsubstituted or is mono- or poly-substituted by lower alkyl, lower alkoxy, lower alkylthio, lower alkanesulfinyl, lower alkanesulfonyl, halo-lower alkyl, halogen and/or by nitro, "lower" radicals being understood as having up to and including seven carbon atoms.

2. A compound according to claim 1 of formula I or Ia, or a salt thereof, in which alk is -CH(R)- and R is hydrogen or lower alkyl.

3. A compound according to claim 1 of formula I or Ia, or a salt thereof, in which the index n is 0, 1 or 2, alk is lower alkylene, especially -CH(R)-, and R is hydrogen or lower alkyl, Ar is phenyl, naphthyl, pyrrolyl, pyrazolyl, imidazolyl, triazolyl, tetrazolyl, furyl, thienyl, isoxazolyl, oxazolyl, oxadiazolyl, isothiazolyl, thiazolyl, thiadiazolyl, pyridyl, pyridazinyl, pyrimidinyl, pyrazinyl or triazinyl, each of which is unsubstituted or is mono- or poly-substituted by lower alkyl, halo-lower alkyl, halogen and/or by nitro, and in corresponding heteroaryl radicals having the -NH group, the hydrogen atom concerned can be substituted by lower alkyl, and the ring A is unsubstituted or is mono- or poly-substituted by lower alkyl, lower alkoxy, lower alkylthio, lower alkanesulfinyl, lower alkanesulfonyl, halo-lower alkyl, halogen and/or by nitro.

4. A compound according to claim 2 of formula I or Ia, or a salt thereof, in which the index n is 0, 1 or 2, Ar is phenyl, naphthyl, pyrrolyl, pyrazolyl, imidazolyl, triazolyl, tetrazolyl, furyl, thienyl, isoxazolyl, oxazolyl, oxadiazolyl, isothiazolyl, thiazolyl, thiadiazolyl, pyridyl, pyridazinyl, pyrimidinyl, pyrazinyl or triazinyl, each

of which is unsubstituted or is mono- or poly-substituted by lower alkyl, halo-lower alkyl, halogen and/or by nitro, the ring A is unsubstituted or is mono- or poly-substituted by lower alkyl, lower alkoxy, lower alkylthio, lower alkanesulfinyl, lower alkanesulfonyl, halo-lower alkyl, halogen and/or by nitro, and alk is -CH(R)- in which R is hydrogen or lower alkyl.

**5.** A compound according to claim 1 of formula I or Ia, or a salt thereof, in which the index n is 0, also 1 or 2, alk is lower alkylene, especially -CH(R)-, and R is hydrogen or lower alkyl, Ar is phenyl, pyrrolyl, 1-lower alkylpyrrolyl, thienyl, thiazolyl or pyridyl, each of which is unsubstituted or is mono- or poly-substituted by lower alkyl, lower alkoxy, halo-lower alkyl, especially trifluoromethyl, or by halogen, and the ring A is unsubstituted or is mono- or poly-substituted by lower alkyl, lower alkoxy, halo-lower alkyl, especially trifluoromethyl, halogen or by nitro.

**6.** A compound according to claim 2 of formula I or Ia, or a salt thereof, in which the index n is 0, 1 or 2, alk is -CH(R)- and R is hydrogen or lower alkyl, Ar is phenyl, pyrrolyl, thienyl or pyridyl, each of which is unsubstituted or is mono- or poly-substituted by halo-lower alkyl, especially trifluoromethyl, or by halogen, and the ring A is unsubstituted or is mono- or poly-substituted by lower alkyl, lower alkoxy, halo-lower alkyl, especially trifluoromethyl, halogen or by nitro.

**7.** A compound according to claim 1 of formula I or Ia, or a salt thereof, in which the index n is 0, alk is -CH(R)- and R is lower alkyl, especially having up to and including 4 carbon atoms, such as methyl, Ar is phenyl, thiazolyl, such as 2-thiazolyl, or pyridyl, such as 2-pyridyl, each of which is unsubstituted or is substituted by lower alkoxy, especially having up to and including 4 carbon atoms, such as methoxy, halogen, especially having an atomic number of up to and including 35, such as fluorine or chlorine, or by trifluoromethyl, in each case localised especially in the 3- or 4-position, or Ar is unsubstituted 1-lower alkylpyrrolyl, especially having up to and including 4 carbon atoms in the lower alkyl moiety, such as 1-methylpyrrol-2-yl, or thienyl, such as 2- or 3-thienyl, which is unsubstituted or is substituted by lower alkyl, especially having up to and including 4 carbon atoms, such as methyl, or halogen, especially having an atomic number of up to and including 35, such as bromine, and the ring A is unsubstituted or is substituted, especially in the 5- or 6-position of the ring system, by halogen, especially having an atomic number of up to and including 35, such as fluorine or chlorine, or by nitro.

**8.** A compound according to claim 2 of formula I or Ia, or a salt thereof, in which the index n is 0, also 1 or 2, alk is -CH(R)- and R is hydrogen or lower alkyl, especially having up to and including 4 carbon atoms, Ar is phenyl that is unsubstituted or is substituted by halolower alkyl, especially having up to and including 2 carbon atoms, such as trifluoromethyl, or by halogen, especially having an atomic number of up to and including 35, such as fluorine or chlorine, or Ar is unsubstituted pyrrolyl, thienyl or pyridyl, and the ring A is unsubstituted or is monosubstituted by halogen, especially having an atomic number of up to and including 35, such as fluorine or chlorine, or by nitro.

**9.** A compound according to claim 2 of formula I or Ia, or a salt thereof, in which the index n is 0, alk is -CH(R)- and R is hydrogen, Ar is phenyl that is unsubstituted or is substituted by trifluoromethyl-or by halogen having an atomic number of up to and including 35, such as fluorine, especially in the 3- or 4-position, or Ar is unsubstituted thienyl, such as 2-thienyl, and the ring A is unsubstituted or is substituted by halogen having an atomic number of up to and including 35, such as fluorine, especially in the 5- or 6-position of the ring system.

**10.** A compound according to claim 1 of formula I or Ia, or a salt thereof, in which the index n is 0, alk is -CH(R)- and R is hydrogen, Ar is-phenyl or 2- or 3-thienyl, and the ring A is unsubstituted or is substituted in the 5- or 6-position of the ring system by fluorine or chlorine.

**11.** A compound according to claim 2 of formula I or Ia, or a salt thereof, in which the index n is 0, alk is -CH(R)- and R is hydrogen, Ar is thienyl, such as 2-thienyl, and the ring A is unsubstituted or is substituted by fluorine, especially in the 5- or 6-position of the ring system.

**12.** 2-hydroxy-3-[2-(2-thienyl)-acetyl]-benzo[b]thiophene or a salt thereof.

**13.** 5-fluoro-2-hydroxy-3-[2-(2-thienyl)-acetyl]-benzo[b]thiophene or a salt thereof.

**14.** 6-fluoro-2-hydroxy-3-[2-(2-thienyl)-acetyl]-benzo[b]thiophene or a salt thereof.

**15.** 5-fluoro-2-hydroxy-3-(2-phenylacetyl)-benzo[b]thiophene or a salt thereof.

**16.** 6-chloro-2-hydroxy-3-[2-(2-thienyl)-acetyl]-benzo[b]thiophene or a salt thereof.

**17.** 5-chloro-2-hydroxy-3-[2-(2-thienyl)-acetyl]-benzo[b]thiophene or a salt thereof.

**18.** 5-fluoro-2-hydroxy-3-[2-(3-thienyl)-acetyl]-benzo[b]thiophene or a salt thereof.

**19.** 2-hydroxy-3-[3-(2-thienyl)-propionyl]-benzo[b]thiophene or a salt thereof.

**20.** A compound according to any one of claims 1 to 19 for use in a method for the treatment of the human or animal body.

**21.** A pharmaceutical composition comprising a compound according to any one of claims 1 to 20 or a pharmaceutically acceptable salt thereof.

**22.** The use of a compound according to any one of claims 1 to 20 for the manufacture of analgesics.

**23.** A process for the preparation of a compound according to any one of claims 1 to 19, in which process:
a) a compound of formula

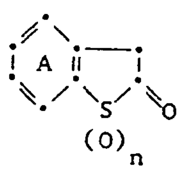

(IIa),

a tautomer or salt thereof, is reacted with a compound of the formula $X_1$-CO-alk-Ar (IIb) in which $X_1$ is reactive esterified hydroxy, or
b) a compound of formula

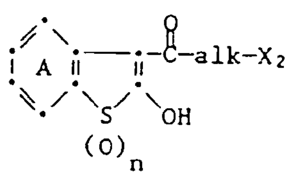

(IIIa),

a tautomer or salt thereof, in which $X_2$ is reactive esterified hydroxy, is reacted with a compound of the formula Ar-H (IIIb),
and, if desired, a compound obtainable in accordance with the process or by other means is converted into a different compound of formula I or Ia, an isomeric mixture obtainable in accordance with the process is separated into the components, a free compound of formula I or Ia obtainable in accordance with the process is converted into a salt and/or a salt obtainable in accordance with the process is converted into the free compound of formula I or Ia or into a different salt.

**Claims for the following Contracting States : ES, GR**

**1.** A process for the preparation of a compound of formula

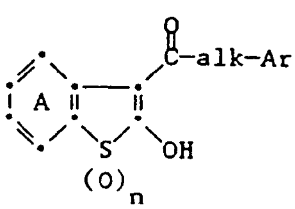

(I) or

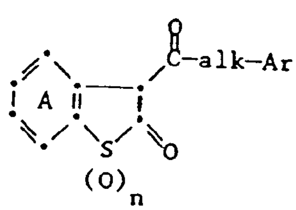

(Ia),

or a salt thereof, in which the index n is 0, 1 or 2, alk is lower alkylene, Ar is phenyl, naphthyl or a monocyclic, five- or six-membered heteroaryl radical, wherein the aromatic radical is unsubstituted or is monoor poly-substituted by lower alkyl, halo-lower alkyl, halogen and/or by nitro, and the ring A is unsubstituted or is mono- or poly-substituted by lower alkyl, lower alkoxy, lower alkylthio, lower alkanesulfinyl, lower alkanesulfonyl, halo-lower alkyl, halogen and/or by nitro, "lower" radicals being understood as having up to and including seven carbon atoms, in which process:

a) a compound of formula

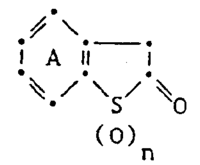

(IIa),

a tautomer or salt thereof, is reacted with a compound of the formula $X_1$-CO-alk-Ar (IIb) in which $X_1$ is reactive esterified hydroxy, or

b) a compound of formula

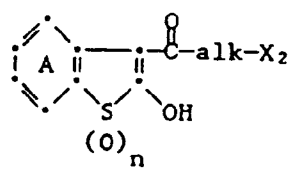

(IIIa),

a tautomer or salt thereof, in which $X_2$ is reactive esterified hydroxy, is reacted with a compound of the formula Ar-H (IIIb),

and, if desired, a compound obtainable in accordance with the process or by other means is converted into a different compound of formula I or Ia, an isomeric mixture obtainable in accordance with the process is separated into the components, a free compound of formula I or Ia obtainable in accordance with the process is converted into a salt and/or a salt obtainable in accordance with the process is converted into the free compound of formula I or Ia or into a different salt.

2. A process according to claim 1 for the preparation of a compound of formula I or Ia, or a salt thereof, in which alk is -CH(R)- and R is hydrogen or lower alkyl.

3. A process according to claim 1 for the preparation of a compound of formula I or Ia, or a salt thereof, in which the index n is 0, 1 or 2, alk is lower alkylene, especially -CH(R)-, and R is hydrogen or lower alkyl, Ar is phenyl, naphthyl, pyrrolyl, pyrazolyl, imidazolyl, triazolyl, tetrazolyl, furyl, thienyl, isoxazolyl, oxazolyl, oxadiazolyl, isothiazolyl, thiazolyl, thiadiazolyl, pyridyl, pyridazinyl, pyrimidinyl, pyrazinyl or triazinyl, each of which is unsubstituted or is mono- or poly-substituted by lower alkyl, halo-lower alkyl, halogen and/or by nitro, and in corresponding heteroaryl radicals having the -NH group, the hydrogen atom concerned can be substituted by lower alkyl, and the ring A is unsubstituted or is mono- or poly-substituted by lower alkyl, lower alkoxy, lower alkylthio, lower alkanesulfinyl, lower alkanesulfonyl, halo-lower alkyl, halogen and/or by nitro.

4. A process according to claim 2 for the preparation of a compound of formula I or Ia, or a salt thereof, in which the index n is 0, 1 or 2, Ar is phenyl, naphthyl, pyrrolyl, pyrazolyl, imidazolyl, triazolyl, tetrazolyl, furyl, thienyl, isoxazolyl, oxazolyl, oxadiazolyl, isothiazolyl, thiazolyl, thiadiazolyl, pyridyl, pyridazinyl, pyrimidinyl, pyrazinyl or triazinyl, each of which is unsubstituted or is mono- or poly-substituted by lower alkyl, halo-lower alkyl, halogen and/or by nitro, the ring A is unsubstituted or is mono- or poly-substituted by lower alkyl, lower alkoxy, lower alkylthio, lower alkanesulfinyl, lower alkanesulfonyl, halo-lower alkyl, halogen and/or by nitro, and alk is -CH(R)- in which R is hydrogen or lower alkyl.

5. A process according to claim 1 for the preparation of a compound of formula I or Ia, or a salt thereof, in which the index n is 0, also 1 or 2, alk is lower alkylene, especially -CH(R)-, and R is hydrogen or lower alkyl, Ar is phenyl, pyrrolyl, 1-lower alkylpyrrolyl, thienyl, thiazolyl or pyridyl, each of which is unsubstituted

or is mono- or poly-substituted by lower alkyl, lower alkoxy, halo-lower alkyl, especially trifluoromethyl, or by halogen, and the ring A is unsubstituted or is mono- or poly-substituted by lower alkyl, lower alkoxy, halo-lower alkyl, especially trifluoromethyl, halogen or by nitro.

6. A process according to claim 2 for the preparation of a compound of formula I or Ia, or a salt thereof, in which the index n is 0, 1 or 2, alk is -CH(R)- and R is hydrogen or lower alkyl, Ar is phenyl, pyrrolyl, thienyl or pyridyl, each of which is unsubstituted or is mono- or poly-substituted by halo-lower alkyl, especially trifluoromethyl, or by halogen, and the ring A is unsubstituted or is mono- or poly-substituted by lower alkyl, lower alkoxy, halo-lower alkyl, especially trifluoromethyl, halogen or by nitro.

7. A process according to claim 1 for the preparation of a compound of formula I or Ia, or a salt thereof, in which the index n is 0, alk is -CH(R)- and R is lower alkyl, especially having up to and including 4 carbon atoms, such as methyl, Ar is phenyl, thiazolyl, such as 2-thiazolyl, or pyridyl, such as 2-pyridyl, each of which is unsubstituted or is substituted by lower alkoxy, especially having up to and including 4 carbon atoms, such as methoxy, halogen, especially having an atomic number of up to and including 35, such as fluorine or chlorine, or by trifluoromethyl, in each case localised especially in the 3- or 4-position, or Ar is unsubstituted 1-lower alkylpyrrolyl, especially having up to and including 4 carbon atoms in the lower alkyl moiety, such as 1-methylpyrrol-2-yl, or thienyl, such as 2- or 3-thienyl, which is unsubstituted or is substituted by lower alkyl, especially having up to and including 4 carbon atoms, such as methyl, or halogen, especially having an atomic number of up to and including 35, such as bromine, and the ring A is unsubstituted or is substituted, especially in the 5- or 6-position of the ring system, by halogen, especially having an atomic number of up to and including 35, such as fluorine or chlorine, or by nitro.

8. A process according to claim 2 for the preparation of a compound of formula I or Ia, or a salt thereof, in which the index n is 0, also 1 or 2, alk is -CH(R)- and R is hydrogen or lower alkyl, especially having up to and including 4 carbon atoms, Ar is phenyl that is unsubstituted or is substituted by halo-lower alkyl, especially having up to and including 2 carbon atoms, such as trifluoromethyl, or by halogen, especially having an atomic number of up to and including 35, such as fluorine or chlorine, or Ar is unsubstituted pyrrolyl, thienyl or pyridyl, and the ring A is unsubstituted or is monosubstituted by halogen, especially having an atomic number of up to and including 35, such as fluorine or chlorine, or by nitro.

9. A process according to claim 2 for the preparation of a compound of formula I or Ia, or a salt thereof, in which the index n is 0, alk is -CH(R)- and R is hydrogen, Ar is phenyl that is unsubstituted or is substituted by trifluoromethyl or by halogen having an atomic number of up to and including 35, such as fluorine, especially in the 3- or 4-position, or Ar is unsubstituted thienyl, such as 2-thienyl, and the ring A is unsubstituted or is substituted by halogen having an atomic number of up to and including 35, such as fluorine, especially in the 5-or 6-position of the ring system.

10. A process according to claim 1 for the preparation of a compound of formula I or Ia, or a salt thereof, in which the index n is 0, alk is -CH(R)- and R is hydrogen, Ar is phenyl or 2- or 3-thienyl, and the ring A is unsubstituted or is substituted in the 5- or 6-position of the ring system by fluorine or chlorine.

11. A process according to claim 2 for the preparation of a compound of formula I or Ia, or a salt thereof, in which the index n is 0, alk is -CH(R)- and R is hydrogen, Ar is thienyl, such as 2-thienyl, and the ring A is unsubstituted or is substituted by fluorine, especially in the 5- or 6-position of the ring system.

12. A process according to claim 2 for the preparation of a compound 2-hydroxy-3-[2-(2-thienyl)-acetyl]-benzo[b]thiophene or a salt thereof.

13. A process according to claim 2 for the preparation of a compound 5-fluoro-2-hydroxy-3-[2-(2-thienyl)-acetyl]-benzo[b]thiophene or a salt thereof.

14. A process according to claim 2 for the preparation of a compound 6-fluoro-2-hydroxy-3-[2-(2-thienyl)-acetyl]-benzo[b]thiophene or a salt thereof.

15. A process according to claim 2 for the preparation of a compound 5-fluoro-2-hydroxy-3-(2-phenylacetyl)-benzo[b]thiophene or a salt thereof.

16. A process according to claim 2 for the preparation of a compound 6-chloro-2-hydroxy-3-[2-(2-thienyl)-acetyl]-benzo[b]thiophene or a salt thereof.

**17.** A process according to claim 2 for the preparation of a compound 5-chloro-2-hydroxy-3-[2-(2-thienyl)-acetyl]-benzo[b]thiophene or a salt thereof.

**18.** A process according to claim 1 for the preparation of a compound 5-fluoro-2-hydroxy-3-[2-(3-thienyl)-acetyl]-benzo[b]thiophene or a salt thereof.

**19.** A process according to claim 1 for the preparation of a compound 2-hydroxy-3-[3-(2-thienyl)-propionyl]-benzo[b]thiophene or a salt thereof.

**20.** The use of a compound according to any one of claims 1 to 19 for the manufacture of analgesics.

**21.** A process for the preparation of a pharmaceutical composition, which process comprises processing a compound according to any one of claims 1 to 19, or a pharmaceutically acceptable salt thereof, optionally in admixture with customary excipients and additives, to give pharmaceutical compositions.

## Revendications

### Revendications pour les Etats contractants suivants : AT, BE, CH, LI, DE, FR, IT, NL, SE, GB, LU

**1 -** Composés de formules respectives

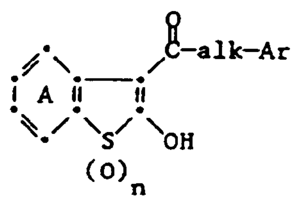 (I) et 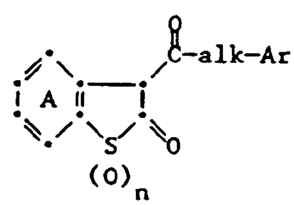 (Ia)

dans lesquelles l'indice n est égal à 0, 1 ou 2, alk représente un groupe alkylène inférieur, Ar représente un groupe phényle, naphtyle ou un groupe hétéroarylé monocyclique à 5 ou 6 chaînons, le groupe aromatique pouvant être non substitué ou porter un ou plusieurs substituants alkyle inférieur, halogénoalkyle inférieur, halogéno et/ ou nitro, et le cycle A étant non substitué ou portant un ou plusieurs substituants alkyle inférieur, alcoxy inférieur, alkylthio inférieur, alcane-sulfinylé inférieur, alcane-sulfonyle inférieur, halogénoalkyle inférieur, halogéno et/ou nitro, l'expression "inférieur" s'appliquant à des groupes qui contiennent jusqu'à 7 atomes de carbone inclus, et leurs sels.

**2 -** Composés selon revendication 1, de formules I et Ia, et leurs sels, pour lesquels alk représente un groupe -CH(R)- et R l'hydrogène ou un groupe alkyle inférieur.

**3 -** Composés selon la revendication 1, de formules I et Ia, et leurs sels, pour lesquels l'indice n est égal à 0, 1 ou 2, alk représente un groupe alkylène inférieur, en particulier un groupe -CH(R)- et R l'hydrogène ou un groupe alkyle inférieur, Ar représente dans chaque cas un groupe phényle, naphtyle, pyrrolyle, pyrazolyle, imidazolyle, triazolyle, tétrazolyle, furyle, thiényle, isoxazolyle, oxazolyle, oxadiazolyle, isothiazolyle, thiazolyle, thiadiazolyle, pyridyle, pyridazinyle, pyrimidinyle, pyrazinyle ou triazinyle non substitué ou portant un ou plusieurs substituants alkyle inférieur, halogénoalkyle inférieur, halogéno et/ou nitro, les groupes hétéroaryle en question portant un groupe -NH- pouvant avoir dans ce groupe l'atome d'hydrogène remplacé par un groupe alkyle inférieur, et le cycle A est non substitué ou porte un ou plusieurs substituants alkyle inférieur, alcoxy inférieur, alkylthio inférieur, alcane-sulfinyle inférieur, alcanesulfonyle inférieur, halogénoalkyle inférieur, halogéno et/ou nitro.

**4 -** Composés selon revendication 2, de formules I et Ia, et leurs sels, pour lesquels l'indice n est égal à 0, 1 ou 2, Ar représente dans chaque cas un groupe phényle, naphtyle, pyrrolyle, pyrazolyle, imidazolyle, triazolyle,tétrazolyle, furyle, thiényle, isoxazolyle, oxazolyle, oxadiazolyle, isothiazolyle, thiazolyle, thiadiazolyle, pyridyle, pyridazinyle, pyrimidinyle, pyrazinyle ou triazinyle non substitué ou portant un ou plusieurs substituants alkyle inférieur, halogénoalkyle inférieur, halogéno et/ou nitro, le cycle A est non substitué ou porte un

ou plusieurs substituants alkyle inférieur, ------- alcoxy inférieur, alkylthio inférieur, alcane-sulfinyle inférieur, alcane-sulfonyle inférieur, halogénoalkyle inférieur, halogéno et/ou nitro, et alk représente -CH(R)- dans lequel R représente l'hydrogène ou un groupe alkyle inférieur.

**5 -** Composés selon revendication 1 de formules I et Ia et leurs sels, pour lesquels l'indice n est égal à 0, ou encore à 1 ou 2, alk représente un groupe alkylène inférieur, en particulier un groupe -CH(R)- dans lequel R représente l'hydrogène ou un groupe alkyle inférieur, Ar représente un groupe phényle, pyrrolyle, 1-(alkyle inférieur)-pyrrolyle, thiényle, thiazolyle ou pyridyle non substitué ou portant un ou plusieurs substituants alkyle inférieur, alcoxy inférieur, halogénoalkyle inférieur, en particulier trifluorométhyle, ou halogéno, et le cycle A est non substitué ou porte un ou plusieurs substituants alkyle inférieur, alcoxy inférieur, halogénoalkyle inférieur, en particulier trifluorométhyle, halogéno ou nitro.

**6 -** Composés selon revendication 2, de formules I et Ia et leurs sels, pour lesquels l'indice n est égal à 0, 1 ou 2, alk représente -CH(R)- et R l'hydrogène ou un groupe alkyle inférieur, Ar représente un groupe phényle, pyrrolyle, thiényle ou pyridyle non substitué ou portant un ou plusieurs substituants halogénoalkyle inférieur, en particulier trifluorométhyle, ou halogéno, et le cycle A est non substitué ou porte un ou plusieurs substituants alkyle inférieur, alcoxy inférieur, halogénoalkyle inférieur, en particulier trifluorométhyle, halogéno ou nitro.

**7 -** Composés selon revendication 1, de formules I et Ia, et leurs sels, pour lesquels l'indice n est égal à 0, alk représente -CH(R)- et R un groupe alkyle inférieur contenant en particulier jusqu'à 4 atomes de carbone inclus, tels que méthyle, Ar représente un groupe phényle, thiazolyle tel que 2-thiazolyle ou pyridyle tel que 2-pyridyle non substitué ou substitué par un groupe alcoxy inférieur, contenant en particulier jusqu'à 4 atomes de carbone inclus, tel que méthoxy, par un halogène, en particulier un halogène de numéro atomique allant jusqu'à 35 inclus, tel que le fluor ou le chlore, ou par un groupe trifluorométhyle, plus spécialement en position 3 ou 4 dans chaque cas, ou bien un groupe 1-(alkyle inférieur)-pyrrolyle non substitué et contenant en particulier jusqu'à 4 atomes de carbone inclus dans la partie alkyle inférieure, par exemple 1-méthylpyrrole-2-yle, ou bien un groupe thiényle tel que 2- ou 3-thiényle, non substitué ou substitué par un groupe alkyle inférieur contenant en particulier jusqu'à 4 atomes de carbone inclus, tel que méthyle, ou par un halogène, en particulier un halogène de numéro atomique allant jusqu'à 35 inclus, tel que le brome, et le cycle A est non substitué ou substitué par un halogène, en particulier un halogène de numéro atomique allant jusqu'à 35 inclus, tel que le fluor ou le chlore, ou par un groupe nitro, en particulier en position 5 ou 6 du système cyclique.

**8 -** Composés selon revendication 2, de formules I et Ia et leurs sels, pour lesquels l'indice n est égal à 0 ou bien encore à 1 ou 2, alk représente -CH(R)- et R l'hydrogène ou un groupe alkyle inférieur contenant en particulier jusqu'à 4 atomes de carbone inclus, Ar représente un groupe phényle non substitué ou substitué par un groupe halogénoalkyle inférieur contenant en particulier jusqu'à 2 atomes de carbone inclus, tel que trifluorométhyle, ou par un halogène, en particulier un halogène de numéro atomique allant jusqu'à 35 inclus, tel que le fluor ou le chlore, ou un groupe pyrrolyle, thiényle ou pyridyle non substitué, et le cycle A est non substitué ou monosubstitué par un halogène, en particulier un halogène de numéro atomique allant jusqu'à 35 inclus, tel que le fluor ou le chlore, ou par un groupe nitro.

**9 -** Composés selon revendication 2, de formules I et Ia et leurs sels, pour lesquels l'indice n est égal à 0, alk représente -CH(R)- et R l'hydrogène, Ar représente un groupe phényle non substitué ou substitué par un groupe trifluorométhyle ou par un halogène de numéro atomique allant jusqu'à 35 inclus, tel que le fluor, en particulier en position 3 ou 4, ou un groupe thiényle non substitué tel que 2-thiényle, et le cycle A est non substitué ou substitué par un halogène de numéro atomique allant jusqu'à 35 inclus, tel que le fluor, en particulier en position 5 ou 6 du système cyclique.

**10 -** Composés selon revendication 1, de formules I et Ia et leurs sels, pour lesquels l'indice n est égal à 0, alk représente -CH(R)- et R l'hydrogène, Ar représente un groupe phényle ou 2- ou 3-thiényle et le cycle A est non substitué ou substitué en position 5 ou 6 du système cyclique par le fluor ou le chlore.

**11 -** Composés selon revendication 2, de formules I et Ia et leurs sels, pour lesquels l'indice n est égal à 0, alk représente -CH(R)- et R l'hydrogène, Ar représente un groupe thiényle tel que 2-thiényle, et le cycle A est non substitué ou substitué par le fluor, en particulier en position 5 ou 6 du système cyclique.

**12 -** Le 2-hydroxy-3-[2-(2-thiényl)-acétyl]-benzo[b]thiophène ou l'un de ses sels.

**13 -** Le 5-fluoro-2-hydroxy-3-[2-(2-thiényl)acétyl]-benzo[b]thiophène ou l'un de ses sels.

**14 -** Le 6-fluoro-2-hydroxy-3-[2-(2-thiényl)acétyl]-benzo[b]thiophène ou l'un de ses sels.

**15 -** Le 5-fluoro-2-hydroxy-3-(2-phénylacétyl)-benzo[b]thiophène ou l'un de ses sels.

**16 -** Le 6-chloro-2-hydroxy-3-[2-(2-thiényl)acétyl]-benzo[b]thiophène ou l'un de ses sels.

**17 -** Le 5-chloro-2-hydroxy-3-[2-(2-thiényl)acétyl]-benzo[b]thiophène ou l'un de ses sels.

**18 -** Le 5-fluoro-2-hydroxy-3-[2-(3-thiényl)acétyl]-benzo[b]thiophène ou l'un de ses sels.

**19 -** Le 2-hydroxy-3-[3-(2-thiényl)-propionyl]-benzo[b]thiophène ou l'un de ses sels.

**20 -** Composés selon l'une des revendications 1 à 19 pour l'utilisation dans un procédé pour le traitement de l'organisme humain ou animal.

**21** - Compositions pharmaceutiques contenant un composé selon l'une des revendications 1 à 20 ou l'un de ses sels acceptables pour l'usage pharmaceutique.

**22** - Utilisation des composés selon une des revendications 1 à 20 pour la préparation de médicaments analgésiques.

**23** - Procédé de préparation des composés selon l'une des revendications 1 à 19, caractérisé en ce que

a) on fait réagir un composé de formule

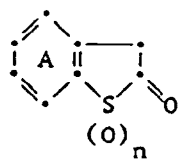

(IIa),

ou un tautomère ou un sel d'un tel composé, avec un composé de formule $X_1$-CO-alk-Ar (IIb) dans laquelle $X_1$ représente un groupe hydroxy estérifié réactif, ou bien

b) on fait réagir un composé de formule

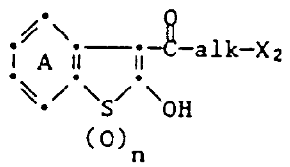

(IIIa),

ou un tautomère ou un sel d'un tel composé, pour lequel $X_2$ représente un groupe hydroxy estérifié réactif, avec un composé de formule Ar-H (IIIb),

et si on le désire on convertit un composé obtenu comme décrit ci-dessus ou par un autre moyen en un autre composé de formule I ou Ia, on sépare un mélange d'isomères obtenu comme décrit ci-dessus en les composants, on convertit un composé de formule I ou Ia obtenu à l'état libre comme décrit ci-dessus en un sel et/ou un sel obtenu comme décrit ci-dessus en le composé libre de formule I ou Ia ou en un autre sel.

**Revendications pour les Etats contractants suivants : ES, GR**

**1** - Procédé de préparation des composés de formules respectives

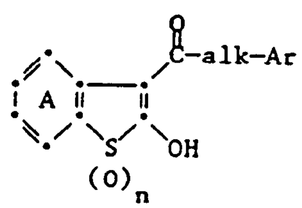

(I) et

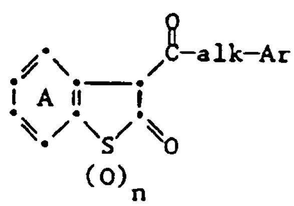

(Ia)

et de leurs sels, pour lesquels n est égal à 0, 1 ou 2, alk représente un groupe alkylène inférieur, Ar représente un groupe phényle, naphtyle ou un groupe hétéroaryle monocyclique à 5 ou 6 chaînons, le groupe aromatique pouvant être non substitué ou porter un ou plusieurs substituants alkyle inférieur, halogénoalkyle inférieur, halogéno- et/ou nitro, et le cycle A est non substitué ou porte un ou plusieurs substituants alkyle inférieur, alcoxy inférieur, alkylthio inférieur, alcane-sulfinyle inférieur, alcane-sulfonyle inférieur, halogénoalkyle inférieur, halogéno et/ou nitro, l'expression "inférieur" s'appliquant à des groupes qui contiennent jusqu'à 7 atomes de carbone inclus, caractérisé en ce que

a) on fait réagir un composé de formule

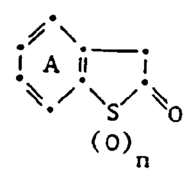

(IIa),

ou un tautomère ou un sel d'un tel composé, avec un composé de formule $X_1$-CO-alk-Ar (IIb) dans laquelle $X_1$ représente un groupe hydroxy estérifié réactif, ou bien

b) on fait réagir un composé de formule

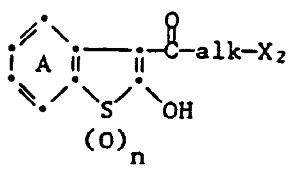

(IIIa),

ou un tautomère ou un sel d'un tel composé, pour lequel $X_2$ représente un groupe hydroxy estérifié réactif, avec un composé de formule Ar-H (IIIb),

et si on le désire, on convertit un composé obtenu comme décrit ci-dessus ou par un autre moyen en un autre composé de formule I ou Ia, on sépare un mélange d'isomères obtenu comme décrit ci-dessus en les composants, on convertit un composé de formule I ou Ia obtenu à l'état libre comme décrit ci-dessus en un sel et/ou un sel obtenu comme décrit ci-dessus en le composé libre de formule I ou Ia ou en un autre sel.

**2 -** Procédé selon revendication 1 pour la préparation des composés de formules I et IIa et de leurs sels pour lesquels alk représente -CH(R)- et R l'hydrogène ou un groupe alkyle inférieur.

**3 -** Procédé selon la revendication 1 pour la préparation des composés de formules I et Ia et de leurs sels pour lesquels l'indice n est égal à 0, 1 ou 2, alk représente un groupe alkylène inférieur, en particulier un groupe -CH(R)- et R l'hydrogène ou un groupe alkyle inférieur, Ar représente dans chaque cas un groupe phényle, naphtyle, pyrrolyle, pyrazolyle, imidazolyle, triazolyle, tétrazolyle, furyle, thiényle, isoxazolyle, oxazolyle, oxadiazolyle, isothiazolyle, thiazolyle, thiadiazolyle, pyridyle, pyridazinyle, pyrimidinyle, pyrazinyle ou triazinyle non substitué ou portant un ou plusieurs substituants alkyle inférieur, halogénoalkyle inférieur, halogéno et/ou nitro, les groupes hétéroarylé en question portant un groupe -NH- pouvant avoir dans ce groupe l'atome d'hydrogène remplacé par un groupe alkyle inférieur, et le cycle A est non substitué ou porte un ou plusieurs substituants alkyle inférieur, alcoxy inférieur, alkylthio inférieur, alcane-sulfinylé inférieur, alcane-sulfonyle inférieur, halogénoalkyle inférieur, halogéno et/ou nitro.

**4 -** Procédé selon la revendication 2 pour la préparation des composés de formules I et Ia et de leurs sels pour lesquels l'indice n est égal à 0, 1 ou 2, Ar représente dans chaque cas un groupe phényle, naphtyle, pyrrolyle, pyrazolyle, imidazolyle, triazolyle, tétrazolyle, furyle, thiényle, isoxazolyle, oxazolyle, oxadiazolyle, isothiazolyle, thiazolyle, thiadiazolyle, pyridyle, pyridazinyle, pyrimidinyle, pyrazinyle ou triazinyle non substitué ou portant un ou plusieurs substituants alkyle inférieur, halogénoalkyle inférieur, halogéno et/ou nitro, le cycle A est non substitué ou porte un ou plusieurs substituants alkyle inférieur, alcoxy inférieur, alkylthio inférieur, alcane-sulfinylé inférieur, alcane-sulfonyle inférieur, halogénoalkyle inférieur, halogéno et/ou nitro, et alk représente -CH(R)- dans lequel R représente l'hydrogène ou un groupe alkyle inférieur.

**5 -** Procédé selon la revendication 1, pour la préparation des composés de formules I et Ia et de leurs sels pour lesquels n est égal à 0, ou encore à 1 ou 2, alk représente un groupe alkylène inférieur, en particulier un groupe -CH(R)- dans lequel R représente l'hydrogène ou un groupe alkyle inférieur, Ar représente un groupe phényle, pyrrolyle, 1-(alkyle inférieur)-pyrrolyle, thiényle, thiazolyle ou pyridyle non substitué ou portant un ou plusieurs substituants alkyle inférieur, alcoxy inférieur, halogénoalkyle inférieur, en particulier trifluorométhyle, ou halogéno, et le cycle A est non substitué ou porte un ou plusieurs substituants alkyle inférieur, alcoxy inférieur, halogéno-alkyle inférieur, en particulier trifluorométhyle, halogéno ou nitro.

**6 -** Procédé selon la revendication 2 pour la préparation des composés de formules I et Ia et de leurs sels pour lesquels l'indice n est égal à 0, 1 ou 2, alk représente -CH(R)- et R l'hydrogène ou un groupe alkyle inférieur, Ar représente un groupe phényle, pyrrolyle, thiényle ou pyridyle non substitué ou portant un ou plusieurs substituants halogénoalkyle inférieur, en particulier trifluorométhyle, ou halogéno, et le cycle A est non substitué ou porte un ou plusieurs substituants alkyle inférieur, alcoxy inférieur, halogénoalkyle inférieur, en particulier trifluorométhyle, halogéno ou nitro.

**7 -** Procédé selon la revendication 1 pour la préparation des composés de formules I et Ia et de leurs sels pour lesquels l'indice n est égal à 0, alk représente -CH(R)- et R un groupe alkyle inférieur contenant en particulier jusqu'à 4 atomes de carbone inclus, tels que méthyle, Ar représente un groupe phényle, thiazolyle tel que 2-thiazolyle ou pyridyle tel que 2-pyridyle non substitué ou substitué par un groupe alcoxy inférieur, contenant en particulier jusqu'à 4 atomes de carbone inclus, tel que méthoxy, par un halogène, en particulier un halogène de numéro atomique allant jusqu'à 35 inclus, tel que le fluor ou le chlore, ou par un groupe trifluorométhyle, plus spécialement en position 3 ou 4 dans chaque cas, ou bien un groupe 1-(alkyle inférieur)-pyrrolyle non substitué et contenant en particulier jusqu'à 4 atomes de carbone inclus dans la partie alkyle inférieure, par exemple 1-méthylpyrrole-2-yle, ou bien un groupe thiényle tel que 2- ou 3-thiényle, non substitué ou substitué par un groupe alkyle inférieur contenant en particulier jusqu'à 4 atomes de carbone inclus, tel que méthyle, ou par un halogène, en particulier un halogène de numéro atomique allant jusqu'à 35 inclus, tel que le brome, et le cycle A est non substitué ou substitué par un halogène, en particulier un halogène de numéro atomique allant jusqu'à 35 inclus, tel que le fluor ou le chlore, ou par un groupe nitro, en particulier en position 5 ou 6 du système cyclique.

**8 -** Procédé selon la revendication 2 pour la préparation des composés de formules I et Ia et de leurs sels pour lesquels l'indice n est égal à 0 ou bien encore à 1 ou 2, alk représente -CH(R)- et R l'hydrogène ou un groupe alkyle inférieur contenant en particulier jusqu'à 4 atomes de carbone inclus, Ar représente un groupe phényle non substitué ou substitué par un groupe halogénoalkyle inférieur contenant en particulier jusqu'à 2 atomes de carbone inclus, tel que trifluorométhyle, ou par un halogène, en particulier un halogène de numéro atomique, allant jusqu'à 35 inclus, tel que le fluor ou le chlore, ou un groupe pyrrolyle, thiényle ou pyridyle non substitué, et le cycle A est non substitué ou monosubstitué par un halogène, en particulier un halogène de numéro atomique allant jusqu'à 35 inclus, tel que le fluor ou le chlore, ou par un groupe nitro.

**9 -** Procédé selon la revendication 2 pour la préparation des composés de formules I et Ia et de leurs sels pour lesquels l'indice n est égal à 0, alk représente -CH(R)- et R l'hydrogène, Ar représente un groupe phényle non substitué ou substitué par un groupe trifluorométhyle ou par un halogène de numéro atomique allant jusqu'à 35 inclus, tel que le fluor, en particulier en position 3 ou 4, ou un groupe thiényle non substitué tel que 2-thiényle, et le cycle A est non substitué ou substitué par un halogène de numéro atomique allant jusqu'à 35 inclus, tel que le fluor, en particulier en position 5 ou 6 du système cyclique.

**10 -** Procédé selon la revendication 1 pour la préparation des composés de formules I et Ia et de leurs sels pour lesquels l'indice n est égal à 0, alk représente -CH(R)- et R l'hydrogène, Ar représente un groupe phényle ou 2- ou 3-thiényle et le cycle A est non substitué ou substitué en position 5 ou 6 du système cyclique par le fluor ou le chlore.

**11 -** Procédé selon la revendication 2 pour la préparation des composés de formules I et Ia et de leurs sels gour lesquels l'indice n est égal à 0, alk représente -CH(R)- et R l'hydrogène, Ar représente un groupe thiényle tel que 2-thiényle, et le cycle A est non substitué ou substitué par le fluor, en particulier en position 5 ou 6 du système cyclique.

**12 -** Procédé selon la revendication 2, pour la préparation du 2-hydroxy-3-[2-(2-thiényl)-acétyl]-benzo [b]thiophène ou de l'un de ses sels.

**13 -** Procédé selon revendication 2 pour la préparation du 5-fluoro-2-hydroxy-3-[2-(2-thiényl)-acétyl]-benzo[b]thiophène ou l'un de ses sels.

**14 -** Procédé selon la revendication 2, pour la préparation du 6-fluoro-2-hydroxy-3-[2-(2-thiényl)-acétyl]-benzo[b]thiophène ou l'un de ses sels.

**15 -** Procédé selon la revendication 2, pour la préparation du 5-fluoro-2-hydroxy-3-(2-phénylacétyl)-benzo[b]thiophène ou l'un de ses sels.

**16 -** Procédé selon la revendication 2, pour la préparation du 6-chloro-2-hydroxy-3-[2-(2-thiényl)-acétyl]-benzo[b]thiophène ou l'un de ses sels.

**17 -** Procédé selon la revendication 2, pour la préparation du 5-chloro-2-hydroxy-3-[2-(2-thiényl)-acétyl]-benzo[b]thiophène ou l'un de ses sels.

**18 -** Procédé selon la revendication 2, pour la préparation du 5-fluoro-2-hydroxy-3-[2-(3-thiényl)-acétyl]-benzo[b]thiophène ou l'un de ses sels.

**19 -** Procédé selon la revendication 2, pour la préparation du 2-hydroxy-3-[3-(2-thiényl)-propionyl]-benzo[b]thiophène ou l'un de ses sels.

**20 -** Utilisation de composés selon l'une des revendications 1 à 19 pour la préparation de médicaments analgésiques.

**21 -** Procédé de préparation de compositions pharmaceutiques, caractérisé en ce que l'on met un composé selon l'une des revendications 1 à 19 ou un sel acceptable gour l'usage pharmaceutique d'un tel composé, éventuellement avec mélange de produits auxiliaires et additifs usuels, sous la forme de composition pharmaceutique.